# EUROPEAN PATENT SPECIFICATION

(11) **EP 3 551 747 B1**
(45) Date of publication and mention of the grant of the patent: **21.10.2026**
(21) Application number: 17878437.7
(22) Date of filing: 08.12.2017
(51) Int. Cl.: C12N 5/075, C07K 14/535

(54) **COMPOSITIONS AND METHODS FOR MATURATION OF OOCYTES IN VITRO**
ZUSAMMENSETZUNGEN UND VERFAHREN ZUR IN-VITRO-REIFUNG VON OOZYTEN
COMPOSITIONS ET PROCÉDÉS DE MATURATION D'OVOCYTES IN VITRO

(30) Priority: 09.12.2016 AU 2016905096
(43) Date of publication of application: 16.10.2019
(73) Proprietor: Adelaide University, Adelaide, SA 5000 (AU)
(72) Inventor: NOTTLE, Mark, Brenton, Bibaringa, South Australia 5118 (AU)
(74) Representative: Müller, Christian Stefan Gerd
(86) International application number: PCT/AU2017/051354
(87) International publication number: WO 2018/102881

(56) References cited:
- US-A1- 2002 028 509
- US-A1- 2012 252 119
- ROBERTSON ET AL: "GM-CSF regulation of embryo development and pregnancy", CYTOKINE AND GROWTH FACTOR REVIEWS, ELSEVIER LTD, GB, vol. 18, no. 3-4, 1 June 2007 (2007-06-01), pages 287 - 298, XP022113442, ISSN: 1359-6101, DOI: 10.1016/J.CYTOGFR.2007.04.008
- ECONOMOU, K. ET AL.: "The combination of calcium ionophore A23187 and GM- CSF can safely salvage aged human unfertilized oocytes after ICSI", JOURNAL OF ASSISTED REPRODUCTION AND GENETICS , 2017, vol. 34, no. 1, 14 October 2016 (2016-10-14), pages 33 - 41, XP036169888
- PERALTA, O. ET AL.: "Granulocyte-macrophage colony stimulating factor (GM-CSF) enhances cumulus cell expansion in bovine oocytes", REPRODUCTIVE BIOLOGY AND ENDOCRINOLOGY, vol. 11, no. 1, 2013, pages 55, XP021157999
- CHEONG, S. ET AL.: "Improvement in the blastocyst quality and efficiency of putative embryonic stem cell line derivation from porcine embryos produced in vitro using a novel culturing system", MOLECULAR MEDICINE REPORTS, vol. 12, no. 2, 16 April 2015 (2015-04-16), pages 2140 - 2148, XP055508940, Retrieved from the Internet <URL:https://www.spandidos-publications.com/mmr/12/2/2140>
- SONDIJ, F. ET AL.: "Abstract P46: Effect of GM-CSF and IL -6 on meiotic resumption of bovine oocytes in vitro and their subsequent fertilization and developmental competence", REPRODUCTION IN DOMESTIC ANIMALS, vol. 39, no. 4, August 2004 (2004-08-01), pages 274, XP055508943
- CUI, X.-S. ET AL.: "Mouse granulocyte-macrophage colony-stimulating factor enhances viability of porcine embryos in defined culture conditions", ANIMAL REPRODUCTION SCIENCE, vol. 84, no. 1, 1 August 2004 (2004-08-01), pages 169 - 177, XP055508946
- GILCHRIST, R. ET AL.: "Effect of granulocyte-macrophage colony-stimulating factor deficiency on ovarian follicular cell function", JOURNAL OF REPRODUCTION AND FERTILITY, vol. 120, 4 January 2000 (2000-01-04), pages 283 - 292, XP055508947

## Description

### PRIORITY CLAIM

This application claims priority from Australian provisional patent application number 2016905096 filed on 9 December 2016.

### FIELD OF THE INVENTION

The present invention relates generally to methods for the maturation of oocytes. In particular, the present invention relates to *in vitro* methods that utilise an improved maturation medium, which increases maturation of oocytes prior to fertilisation.

### BACKGROUND OF THE INVENTION

In mammals, immature eggs (oocytes) grow and develop in follicles within the ovary. Immature oocytes are metabolically coupled to somatic granulosa cells, which surround the oocyte and nurture the development of the oocyte until ovulation. Essentially, maturation of the oocyte depends on its association with its companion somatic granulosa cells (termed cumulus cells once the oocyte is ovulated) which not only support its growth and development, but also regulate the progression of meiosis.

The cytoplasmic and nuclear maturation of the oocyte during pre-ovulatory development are closely related but differentially distinguishable processes crucial for successful fertilisation, development of the embryo, and also for the ability of the embryo to implant, develop to term and produce healthy offspring.

During cytoplasmic development, the diameter of the oocyte substantially increases from ~15 to 100 µm, corresponding to a 300-fold increase in volume. At this stage the oocyte is both transcriptionally and translationally very active. For example, a mature mouse oocyte contains ~200-fold more RNA and ~50-60-fold more protein than an average somatic cell. The content of mRNA in the oocyte is also high, -15-20%, compared to that of ~2-3% in a somatic cell.

Nuclear maturation of the oocyte occurs after the gonadotropin luteinising hormone surge, and involves the dissolution of the nuclear membrane, chromosome condensation followed by orientation in the equatorial plate, and organisation of the microtubules in a spindle.

A significant proportion of children in western countries are now born using assisted reproduction technologies, including the use of *in vitro* fertilization (IVF). IVF generally takes the form of hormonally stimulating the ovaries of women to produce multiple growing follicles, collecting the ova from these preovulatory follicles, fertilizing collected ova with sperm *in vitro* and introducing the resultant embryo into the uterus. Given that a number of these steps occur outside of the reproductive tract, mimicking the natural environment of the oocyte is an important consideration towards maximizing the success of an IVF program.

Large doses of gonadotropin, or other ovarian follicle stimulating agents used in standard IVF procedures, can lead to a condition of ovarian hyperstimulation syndrome (OHSS), which occurs in approximately 5% of women undertaking IVF cycles. OHSS is usually mild and self-limiting. In some cases, urgent medical attention is required. When severe, the condition can be potentially life threatening requiring hospitalization, intravenous fluids, pain relief, and other medication. Pulmonary embolism from a clot in the leg or complications of severe dehydration may occur in rare cases.

*In vitro* maturation (IVM) of oocytes prior to fertilization is increasingly used an adjunct therapy to IVF, which greatly reduces the requirement for hormone administration during treatment. IVM involves the removal of ova from smaller follicles in patients who receive either low levels of gonadotropin or even no gonadotropin. The procedure used to obtain eggs requires a modified patient management system and ova pick-up procedure.

Women with the condition of polycystic ovarian syndrome require IVM in preference to IVF to avoid ovarian hyperstimulation caused by the administration of gonadotropin, or other ovarian follicle stimulating agents. IVM is also applied to women who have been advised to minimize follicle stimulation during infertility treatment, and is used in female cancer patients undergoing chemotherapy and who are requiring fertility preservation. IVM is also more convenient to the patient as it requires less drug administration, which is usually performed by the patients themselves. Therefore, IVM has cost advantages, as the cost of drug use is minimized.

Nevertheless, the efficiency of IVM relative to IVF in establishing pregnancies and live births are reduced. Although there have been some improvements in recent times to patient management, there has been little advance in laboratory techniques despite the fact that current IVM methods do not replicate the environment in the maturing follicle and reproductive tract.

In non-human mammals, *in vitro* production (IVP) of animal embryos has a variety of purposes, such as genetic improvement in livestock and domesticated breeds, genetic rescue in rarer breeds, as well as a platform technology for manipulations, such as production of sexed embryos from sexed sperm, or cloning by somatic cell nuclear transfer. An essential technique in the production of embryos *in vitro* is the maturation of oocytes *in vitro.* IVP has the potential to replace current conventional techniques such as multiple ovulation and embryo transfer (MOET), where (similarly to human clinical application) gonadotropin treatment is required. However, adoption of IVP for breeding and other uses has been hampered by the relatively low efficiencies of producing transferable stage embryos, the poor results following embryo transfer of such embryos and the poor results following freezing and thawing (storage) of such embryos. The immaturity of oocytes used for IVP of animal embryos is reflective of these poor results.

Sondij F et al., Reproduction in Domestic Animals, 39 (2004), page 274, abstract P46, describes the effect of 0.1, 1.0 and 10 ng/ml of recombinant human GM-CSF on bovine oocyte maturation.

Accordingly, new compositions and methods for maturing oocytes *in vitro,* for example to improve the effectiveness of assisted reproductive technologies, are highly desirable.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application.

### SUMMARY OF THE INVENTION

The present invention is defined by the appended claims.

The present invention provides a method of increasing maturation of a mammalian oocyte *in vitro,* the method comprising culturing the oocyte *in vitro* in a mammalian oocyte maturation medium comprising 0.1 ng/ml to 100 ng/ml species-specific granulocyte macrophage-colony stimulating factor (GM-CSF).

In one embodiment of the method, the GM-CSF increases developmental competence of the oocyte.

In one embodiment of the method, the mammalian oocyte is selected from the group consisting of a human oocyte, a bovine oocyte, a porcine oocyte, an equine oocyte, a canine oocyte, a feline oocyte, a murine oocyte, an ovine oocyte, and a non-human primate oocyte.

In one embodiment of the method, the mammalian oocyte is from an aged and/or obese subject.

### SUMMARY OF THE DESCRIPTION

The following description may on some occasions extend beyond the scope of the invention (the claims). In particular, the term "embodiment" is not to be construed as necessarily referring to the claimed invention, unless the subject-matter in question falls within the scope of the claims. In the context of the present specification, in vitro fertilization and the subsequent in vitro embryo production are disclosed for reference only and do not form part of the claimed invention.

The present invention is predicated, in part, on the finding that granulocyte macrophage-colony stimulating factor (GM-CSF) is effective at increasing the maturation and developmental competence of an oocyte *in vitro.* This enables the formulation and preparation of media for use in *in vitro* maturation of oocytes.

Accordingly, in a first aspect, described herein is an *in vitro* oocyte maturation medium, the medium comprising granulocyte macrophage-colony stimulating factor (GM-CSF).

In a second aspect, described herein is a medium for increasing maturation of an oocyte *in vitro,* the medium comprising granulocyte macrophage-colony stimulating factor (GM-CSF).

In a third aspect, described herein is an *in vitro* culture medium when used for increasing maturation of an oocyte, the medium comprising granulocyte macrophage-colony stimulating factor (GM-CSF).

In some embodiments of the first to third aspects, the GM-CSF increases the developmental competence of an oocyte cultured in the medium. Developmental competence may be measured, for example, *in vitro* by an increase in cleavage rate, on time blastocyst development and/or development to the blastocysts stage.

As descrbed herein, an embryo derived from an oocyte cultured in the medium has one or more of an increased blastocyst inner cell mass number, increased blastocyst rate, increased trophectoderm cell number, increased blastocyst total cell number, and increased viability.

As described herein, a blastocyst derived from an oocyte cultured in the medium has decreased DNA damage. A decrease in DNA damage may in turn reduce or normalize abherrant gene expression, which may be a consequence of *in vitro* culture.

As described herein, one or more of implantation, pregnancy rate, and development to term in a surrogate following transfer of an embryo derived from an oocyte cultured in the medium to the surrogate is increased.

In some embodiments of the aforementioned aspects, the oocyte is selected from the group consisting of a human oocyte, a bovine oocyte, a porcine oocyte, an equine oocyte, a canine oocyte, a feline oocyte, a murine oocyte, an ovine oocyte, and a non-human primate oocyte.

In some embodiments of the aforementioned aspects, the oocyte is from an aged and/or obese subject.

In some embodiments of the aforementioned aspects, the GM-CSF is species-specific. In some embodiments, the amount of GM-CSF present in the medium is about 0.1 ng/ml to about 100 ng/ml.

In a fourth aspect, described herein is a method of *in vitro* maturation of an oocyte, the method comprising culturing the oocyte in a medium of any one of the first to third aspects of the invention.

In a fifth aspect, described herein is an oocyte matured by the method of the fourth aspect.

In a sixth aspect, described herein is an embryo or non-human animal produced from the oocyte of the fifth aspect.

In a seventh aspect, described herein is a method of increasing maturation of an oocyte *in vitro,* the method comprising culturing the oocyte in a medium comprising granulocyte macrophage-colony stimulating factor (GM-CSF). According to the present invention, the method of increasing maturation of a mammalian oocyte *in vitro* comprises culturing the oocyte *in vitro* in a mammalian oocyte maturation medium comprising 0.1 ng/ml to 100 ng/ml species-specific granulocyte macrophage-colony stimulating factor (GM-CSF).

In an eighth aspect, described herein is a method of increasing developmental competence of an oocyte *in vitro,* the method comprising culturing the oocyte in a medium comprising granulocyte macrophage-colony stimulating factor (GM-CSF).

As described herein, an embryo derived from the oocyte has one or more of an increased blastocyst inner cell mass number, increased blastocyst rate, increased trophectoderm cell number, increased blastocyst total cell number, and increased viability.

As described herein, a blastocyst derived from an oocyte cultured in the medium has decreased DNA damage.

As described herein, one or more of implantation, pregnancy rate, and development to term, in a surrogate following transfer of an embryo derived from an oocyte cultured in the medium to the surrogate is increased.

As described herein, the method is used as part of an assisted reproductive technology.

In some embodiments of the seventh and eighth aspects, the oocyte is selected from the group consisting of a human oocyte, a bovine oocyte, a porcine oocyte, an equine oocyte, a canine oocyte, a feline oocyte, a murine oocyte, an ovine oocyte, and a non-human primate oocyte.

In some embodiments of the seventh and eighth aspects, the oocyte is from an aged and/or obese subject.

In some embodiments of the seventh and eighth aspects, the GM-CSF is species-specific. In some embodiments, the amount of GM-CSF present in the medium is about 0.1 ng/ml to about 100 ng/ml. According to the present invention, the GM-CSF is species-specific, and the amount of GM-CSF present in the medium is 0.1 ng/ml to 100 ng/ml.

As described herein, described herein is a method of producing an embryo from an oocyte by an assisted reproductive technology, the method comprising:
(a) collecting an oocyte from an ovary of a subject;
(b) culturing the oocyte *in vitro* in a medium comprising GM-CSF; and
(c) producing an embryo from the oocyte by fertilising the oocyte *in vitro.*

As described herein, one or more of implantation, pregnancy rate, and development to term, in a surrogate following transfer of the embryo to the surrogate is increased.

In some embodiments of the ninth aspect, the oocyte is collected from an aged and/or obese subject.

As described herein, sperm used in the assisted reproductive technology is cultured in a medium comprising GM-CSF prior to fertilisation of the oocyte.

As described herein, fertilisation of the oocyte takes place in a medium comprising GM-CSF.

As described herein, the method further comprises the step of culturing the embryo once produced in a medium comprising GM-CSF.

Described herein is a method of assisted reproduction involving an oocyte, the method comprising the step of culturing the oocyte *in vitro* in a medium comprising GM-CSF.

Described herein is a method of assisted reproduction involving an oocyte, the method comprising the step of *in vitro* maturation of an oocyte by culturing the oocyte *in vitro* in a medium comprising GM-CSF.

As described herein, the method comprises the further step of fertilizing the oocyte *in vitro* with sperm that has been cultured in a medium comprising GM-CSF.

As described herein, the method comprises the further step of culturing an embryo derived from the oocyte *in vitro* in a medium comprising GM-CSF. In some embodiments, one or more of implantation, pregnancy rate, and development to term, in a surrogate following transfer of the embryo to the surrogate is increased.

In some embodiments of the tenth and eleventh aspects, the oocyte is collected from an aged and/or obese subject.

In a twelfth aspect, described herein is a granulocyte macrophage-colony stimulating factor (GM-CSF) for use, or when used, in a culture medium for increasing maturation of an oocyte *in vitro* or for increasing developmental competence of an oocyte *in vitro.*

As described herein, an embryo derived from the oocyte cultured in the culture medium has one or more of an increased blastocyst inner cell mass number, increased blastocyst rate, increased trophectoderm cell number, increased blastocyst total cell number, and increased viability.

As described herein, a blastocyst derived from an oocyte cultured in the medium has decreased DNA damage.

As described herein, the GM-CSF is used in a culture medium as part of an assisted reproductive technology. In one embodiment, the assisted reproductive technology is *in vitro* fertilization.

As described herein, one or more of implantation, pregnancy rate, and development to term in a surrogate following transfer of an embryo derived from an oocyte cultured in the medium to the surrogate is increased.

In some embodiments of the twelfth and thirteenth aspects, the oocyte is selected from the group consisting of a human oocyte, a bovine oocyte, a porcine oocyte, an equine oocyte, a canine oocyte, a feline oocyte, a murine oocyte, an ovine oocyte, and a non-human primate oocyte.

In some embodiments of the twelfth aspect, the oocyte is from an aged and/or obese subject.

In some embodiments of the twelfth and thirteenth aspects, the GM-CSF is species-specific. In some embodiment, the amount of GM-CSF present in the medium is about 0.1 ng/ml to about 100 ng/ml.

In a thirteenth aspect, described herein is a use of a granulocyte macrophage-colony stimulating factor (GM-CSF) in the preparation of a culture medium for increasing maturation of an oocyte *in vitro* or for increasing developmental competence of an oocyte *in vitro.*

As described herein, an embryo derived from the oocyte cultured in the culture medium has one or more of an increased blastocyst inner cell mass number, increased blastocyst rate, increased trophectoderm cell number, increased blastocyst total cell number, and increased viability.

As described herein, a blastocyst derived from an oocyte cultured in the medium has decreased DNA damage.

As described herein, the culture medium is used as part of an assisted reproductive technology. In one embodiment, the assisted reproductive technology is *in vitro* fertilization.

As described herein, one or more of implantation, pregnancy rate, and development to term in a surrogate following transfer of an embryo derived from an oocyte cultured in the medium to the surrogate is increased.

In some embodiments of the thirteenth aspect, the oocyte is selected from the group consisting of a human oocyte, a bovine oocyte, a porcine oocyte, an equine oocyte, a canine oocyte, a feline oocyte, a murine oocyte, an ovine oocyte, and a non-human primate oocyte.

In some embodiments of the thirteenth aspect, the oocyte is from an aged and/or obese subject.

In some embodiments of the thirteenth aspect, the GM-CSF is species-specific. In some embodiments, the amount of GM-CSF present in the medium is about 0.1 ng/ml to about 100 ng/ml.

In a fourteenth aspect, described herein is a combination product for use in, or when used for, increasing maturation of an oocyte *in vitro* or for increasing developmental competence of an oocyte *in vitro,* the combination product comprising:
(i) a culture medium;
(ii) granulocyte macrophage-colony stimulating factor (GM-CSF); and optionally
(iii) instructions for culturing an oocyte in the culture medium comprising the GM-CSF.

In a fifteenth aspect, described herein is a combination product for use in, or when used for, increasing maturation of an oocyte *in vitro* or for increasing developmental competence of an oocyte *in vitro,* the combination product comprising:
(i) a culture medium comprising granulocyte macrophage-colony stimulating factor (GM-CSF); and
(ii) instructions for culturing an oocyte in the culture medium.

As described herein, an embryo derived from the oocyte cultured in the culture medium comprising the GM-CSF has one or more of an increased blastocyst inner cell mass number, increased blastocyst rate, increased trophectoderm cell number, increased blastocyst total cell number, and increased viability.

As described herein, a blastocyst derived from an oocyte cultured in the medium has decreased DNA damage.

As described herein, the combination product is used as part of an assisted reproductive technology. In one embodiment, the assisted reproductive technology is *in vitro* fertilization.

As described herein, one or more of implantation, pregnancy rate, and development to term in a surrogate following transfer of an embryo derived from an oocyte cultured in the medium to the surrogate is increased.

In some embodiments of the fourteenth and fifteenth aspects, the oocyte is selected from the group consisting of a human oocyte, a bovine oocyte, a porcine oocyte, an equine oocyte, a canine oocyte, a feline oocyte, a murine oocyte, an ovine oocyte, and a non-human primate oocyte.

In some embodiments of the fourteenth and fifteenth aspects, the oocyte is from an aged and/or obese subject.

In some embodiments of the fourteenth and fifteenth aspects, the GM-CSF is species-specific.

### BRIEF DESCRIPTION OF THE FIGURES

For a further understanding of the aspects and advantages of the present disclosure, reference should be made to the following detailed description, taken in conjunction with the accompanying figures.

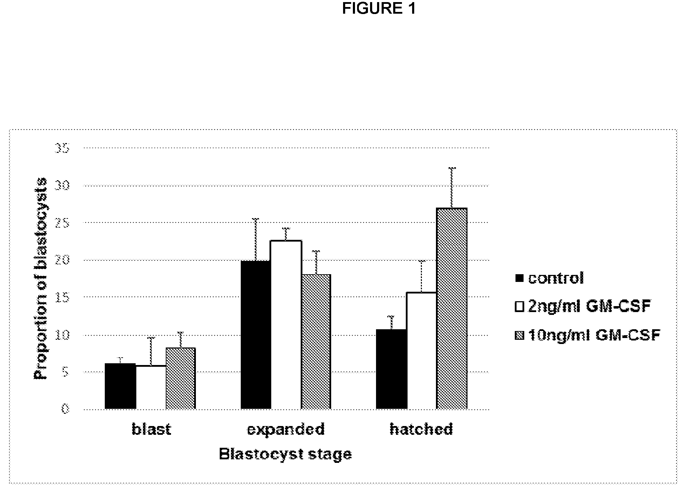

**FIGURE 1** - a graph showing the effect of adding GM-CSF to oocyte maturation media on the proportion of blastocysts that had hatched by day 8 of culture. The addition of 2 ng/ml and 10 ng/ml of bovine GM-CSF to oocyte maturation media increased the proportion of hatched bovine blastocysts on day 8 of embryo culture by 45.4% and 149.0% respectively, compared with a control. 0 ng (control; black), 2 ng/ml GM-CSF (white), and 10 ng/ml GM-CSF (grey). Values mean ± SEM of five replicates.

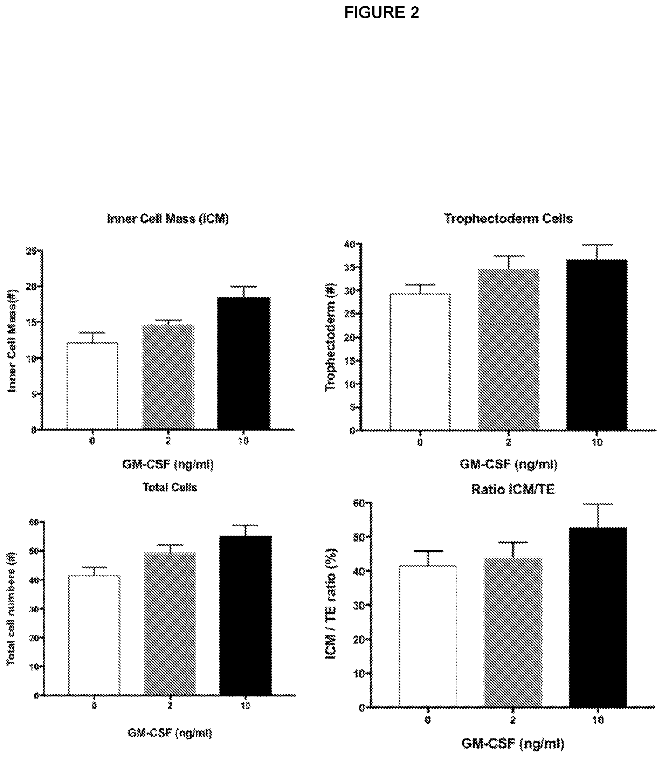

**FIGURE 2** - graphs showing the effect of adding GM-CSF to oocyte maturation media on blastocyst inner cell mass, trophectoderm and total cell numbers. The addition of GM-CSF to oocyte maturation media increased murine blastocyst ICM and total cell numbers by 20.5% and 52.1%, trophectoderm cell numbers by 18.2% and 25.0%, and total cell numbers by 18.8% and 32.9%, respectively. 0 ng (control; white), 2 ng/ml GM-CSF (grey), and 10 ng/ml GM-CSF (black). Values mean ± SEM of six replicates.

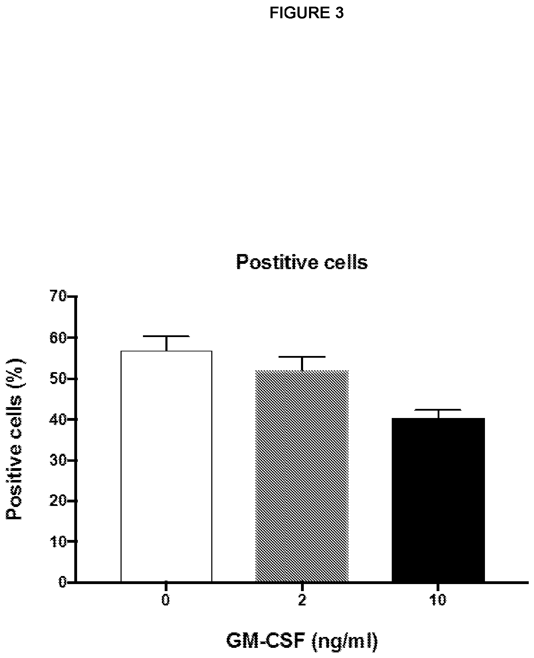

**FIGURE 3** - a graph showing the effect of adding GM-CSF to oocyte maturation media on murine DNA blastocyst damage. The addition of GM-CSF to maturation media reduced the incidence of DNA double strand breaks in the 2 ng/ml GM-CSF and 10 ng/ml GM-CSF groups by 8.4% and 29.1%, respectively, compared with the control. Values are expressed as a percentage and are the mean ± SEM of five replicates.

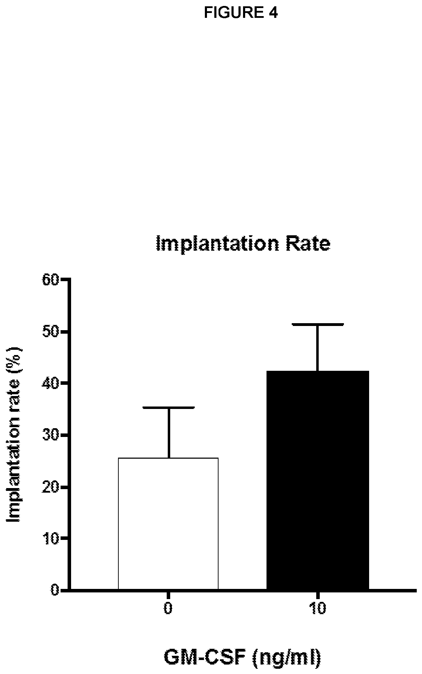

**FIGURE 4** - a graph showing the effect of maturing murine oocytes with GM-CSF on implantation rate. Values are expesed as a percentage of the number of implantation sites/embryos transferred and are the mean ± SEM of 11 transfers.

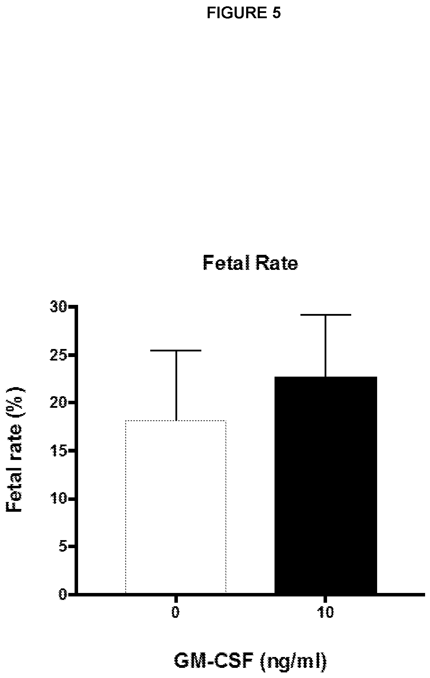

**FIGURE 5** - a graph showing the effect of maturing murine oocytes with GM-CSF on the number of fetuses present at day 17.5 post coitus. Values are expesed as a percentage of fetuses present/embryos transferred and are the mean ± SEM of 11 transfers.

### DETAILED DESCRIPTION

Nucleotide and polypeptide sequences are referred to herein by a sequence identifier number (SEQ ID NO:). A summary of the sequence identifiers is provided in Table 1. A sequence listing has also been provided at the time of filing this application.

**TABLE 1**

| **Summary of sequence identifiers** | |
|---|---|
| **Sequence Identifier** | **Sequence** |
| SEQ ID NO: 1 | Human GM-CSF nucleotide sequence |
| SEQ ID NO: 2 | Human GM-CSF amino acid sequence |
| SEQ ID NO: 3 | Bovine GM-CSF nucleotide sequence |
| SEQ ID NO: 4 | Bovine GM-CSF amino acid sequence |
| SEQ ID NO: 5 | Porcine GM-CSF nucleotide sequence |
| SEQ ID NO: 6 | Porcine GM-CSF amino acid sequence |
| SEQ ID NO: 7 | Equine GM-CSF nucleotide sequence |
| SEQ ID NO: 8 | Equine GM-CSF amino acid sequence |
| SEQ ID NO: 9 | Canine GM-CSF nucleotide sequence |
| SEQ ID NO: 10 | Canine GM-CSF amino acid sequence |
| SEQ ID NO: 11 | Feline GM-CSF nucleotide sequence |
| SEQ ID NO: 12 | Feline GM-CSF amino acid sequence |
| SEQ ID NO: 13 | Murine GM-CSF nucleotide sequence |
| SEQ ID NO: 14 | Murine GM-CSF amino acid sequence |
| SEQ ID NO: 15 | Ovine GM-CSF nucleotide sequence |
| SEQ ID NO: 16 | Ovine GM-CSF amino acid sequence |

The following abbreviations, as used throughout the specification, are defined herein as follows:
- IVF: *In vitro* Fertilization
- OHSS: Ovarian Hyperstimulation Syndrome
- IVM: *In vitro* Maturation
- IVP: *In vitro* Production
- MOET: Multiple Ovulation and Embryo Transfer
- GM-CSF: Granulocyte Macrophage-Colony Stimulating Factor
- HSA: Human Serum Albumin
- ITS: Insulin-Transferrin-Selenium
- IGF-1: Insulin-Like Growth Factor-1
- EGF: Epidermal Growth Factor
- FSH: Follicle Stimulating Hormone
- HCG: Human Chorionic Gonadotropin
- LH: Luteinizing Hormone
- PMSG: Pregnant Mare's Serum Gonadotropin
- ART: Assisted Reproductive Technology
- ICSI: Intracytoplasmic Sperm Injection
- PVA: Polyvinyl Alcohol

As shown herein, inclusion of granulocyte macrophage-colony stimulating factor (herein referred to as "GM-CSF") in a culture medium increases the maturation and developmental competence of an oocyte cultured in the medium *in vitro.* With respect to assisted reproduction technologies, this allows oocytes harvested from the ovary to mature to a point close to that occurring naturally *in vivo* during the reproductive cycle (when compared to the maturity of oocytes collected and cultured in currently known media) prior to subsequent fertilisation of the oocyte *in vitro.*

Certain disclosed embodiments have one or more combinations of advantages. For example, some of the advantages of the embodiments disclosed herein include one or more of the following: culture media and methods for increasing oocyte maturation; culture media and methods for increasing developmental competence of an oocyte; culture media and methods for increasing blastocyst inner cell mass number, increasing blastocyst rate, increasing blastocyst trophectoderm cell number, increasing blastocyst total cell number, and/or increasing viability of an embryo derived from an oocyte cultured in the media; culture media and methods for decreasing DNA damage in a blastocyst derived from an oocyte cultured in the media; culture media and methods for increasing pregnancy rate following transfer of an embryo derived from an oocyte cultured in the medium to a surrogate; culture media and methods for increasing implantation of a blastocyst derived from an oocyte cultured in the medium in a surrogate; culture media and methods for increasing development to term of an embryo derived from an oocyte cultured in the medium; to provide one or more advantages, or to provide a commercial alternative. Other advantages of some embodiments of the present disclosure are provided herein.

Accordingly, in various aspects, described herein is an *in vitro* oocyte maturation medium, a medium for increasing maturation of an oocyte *in vitro,* and/or an *in vitro* culture medium when used for increasing maturation of an oocyte, the medium comprising granulocyte macrophage-colony stimulating factor (GM-CSF).

The terms "maturation medium", "medium for increasing maturation", and "*in vitro* culture medium" are not intended to define different media. In fact, these terms can effectively be used interchangeably and do indeed relate to the same medium, that medium comprising GM-CSF for use in oocyte maturation.

The base medium to which GM-CSF is added may be any medium which supports and maintains the viability of an oocyte cultured in the medium *in vitro.* A suitable base medium for example may include, but is not limited to, Tissue Culture Medium 199 (also known as Media 199, TCM199, and M199)(ThermoFisher Scientific), Minimum Essential Medium Eagle (also known as Eagles' Minimum Essential Medium, EMEM and MEM)(Sigma-Aldrich), Minimum Essential Medium Eagle Alpha Modifications (also known as α-MEM)(Sigma-Aldrich), Dulbecco's Modified Eagle Medium (also known as DMEM or D-MEM)(ThermoFisher Scientific), Ham's F12 (also known as F-12 Ham, Ham's F12 Medium, and F12 Nutrient Mixture)(ThermoFisher Scientific), RPMI Medium 1640 (ThermoFisher Scientific), Isocove's Modified Dulbecco's Medium (also known as IMDM)(ThermoFisher Scientific), Waymouth's MB 752/1 Medium (also known as Waymouth or Waymouth Medium)(Sigma-Aldrich), Chang's Medium (Irvine Scientific), HTF Medium (Irvine Scientific), Dulbecco's Modified Eagle's Medium/Nutrient F-12 Ham (also known as DMEM/F-12 and DME F12)(ThermoFisher Scientific), Vitromat (IVF Vet Solutions, Robinson Research Institute, University of Adelaide, SA, Australia) and ART-1600-B (Origio, Denmark).

With respect to the *in vitro* maturation of human oocytes, companies such as Origio (Denmark) provide appropriate base media. For example, GM-CSF may be added to the ART-1600-B medium supplied by Origio.

Despite the inclusion of the list above, other base media are contemplated, as would be understood by a person skilled in the art, provided that the base media supports and maintains the viability of an oocyte cultured in the medium *in vitro.*

The base medium to which GM-CSF is added may also be supplemented with additional components. For example, see Culture Media, Solutions, and Systems in Human ART (2014), Editor Patrick Quinn, Cambridge University Press, ISBN 978-1-107-61953-1. Examples of additional components include, but are not limited to, inorganic ions (such as cations and anions - Na⁺, K⁺, Mg²⁺, Ca²⁺, Cl⁻, SO₄²⁻, PO₄²⁻, HCO₃⁻), energy substrates (such as glucose, lactate, pyruvate, amino acids), nitrogen sources (such as essential and non-essential amino acids), vitamins, fatty acids or precursors, nucleic acid precursors, chelators (such as EDTA), antioxidants, proteins or macromolecules (such as HSA and hyaluronate), other growth factors or hormones (such as insulin-transferrin-selenium, insulin-like growth factor, epidermal growth factor, follicle stimulating hormone and human chorionic gonadotropin), buffers to maintain a physiological pH, antibiotics, pH indicators, and combinations of any one or more of the above. It is to be made clear that certain base media may already contain one or more of the additional components listed above.

Preferred amounts and ranges of these additional components can be found in standard text books known in the art. For example, see Culture Media, Solutions, and Systems in Human ART (2014, *supra*), and Textbook of Assisted Reproduction: Laboratory and Clinical Perspectives (2003) Editors Gardner, D.K., Weissman, A., Howles, C.M., Shoham, Z. Martin Dunits Ltd, London, UK; and Gordon, I. (2003) Laboratory Production of Cattle Embryos 2nd Edition CABI Publishing, Oxon, UK.

For example, the maturation medium described herein and as used in the method of the present invention may include one or more of the following components in the concentration ranges indicated:

| | | | |
|---|---|---|---|
| NaCl | 80.0-100 mM | KCI | 3.5-7.5 mM |
| NaH₂PO₄•2H₂O | 0.05-1.5 mM | MgSO₄•7H₂O | 0.2-4.0 mM |
| NaHCO₃ | 15- 30 mM | CaCl₂•2H₂O | 0.8- 2.8 mM |
| Glucose | 0.5- 5.5 mM | NaLactate | 2.0- 20 mM |
| NaPyruvate | 0.01-5.0 mM | Alanine | 0.01- 1.0 mM |
| Aspartate | 0.01- 1.0 mM | Asparagine | 0.01- 1.0 mM |
| L-Glutamine | 0.01- 1.0 mM | Alanyl-Glutamine | 0.01- 2.0 mM |
| Glycine | 0.01- 1.0 mM | Proline | 0.01- 1.0 mM |
| Serine | 0.01- 1.0 mM | Cysteamine | 0.1- 2.0 mM |
| L-Arginine-HCl | 0.1-1.2 mM | L-Cystine 2HCl | 0.05- 0.25 mM |
| L-Histidine-HCl-H₂O | 0.1- 0.4 mM | L-Isoleucine | 0.1- 0.8 mM |
| L-Leucine | 0.1- 0.8 mM | L-Lysine-HCl | 0.1- 0.8 mM |
| L-Methionine | 0.05- 0.25 mM | L-Phenylalanine | 0.1- 0.4 mM |
| L-Threonine | 0.1- 0.8 mM | L-Tryptophan | 0.1- 0.9 mM |
| L-Tyrosine 2Na | 0.1- 0.4 mM | L-Valine | 0.1-0.8 mM |
| D-Ca Pantotherate | 0.001- 0.004 mM | Choline Chloride | 0.003-0.01 mM |
| Folic Acid | 0.001-0.0045 mM | i-Inositol | 0.005-0.02 mM |
| Niacinamide | 0.004-0.016 mM | Pyridoxal HCl | 0.002-0.01 mM |
| D-Pantothenic acid | 0.004-0.016 mM | Riboflavin | 0.0001-0.0006 mM |
| Thiamin HCl | 0.001-0.006 mM | HSA | 1-10.0 mg/ml |
| Hyaluronate | 0.05-0.5 mg/ml | ITS | 1-100 ng/ml |
| IGF-1 | 10-1000 ng/ml | EGF | 1-500 ng/ml |
| FSH | 0.001-10 U/ml | HCG | 1-100 U/ml |
| LH | 0.01-10 U/ml | Penicillin | 5.0-500 µg/ml |
| Streptomycin | 5.0-500 µg/ml | Gentamicin | 0.1-10.0 µg/ml |
| Insulin | 0.1-10.0 µg/ml | PMSG | 1-100 U/ml |
| Follicular fluid | 1-20% v/v | Phenol red Na | 0.001-0.1 mg/ml |
| PVA | 1-10 mg/ml | Fetuin | 0.1-10 mg/ml |
| BSA | 1-10 mg/ml | | |

With respect to the maturation of human oocytes, a suitable maturation medium according to an embodiment of the present invention may include the following components in the amounts indicated:

| | | | |
|---|---|---|---|
| NaCl | 6.8 mg/ml | KCI | 400 µg/ml |
| NaH₂PO₄ | 122 µg/ml | MgSO₄•6H₂O | 97.67 µg/ml |
| NaHCO₃ | 2.2 mg/ml | CaCl₂•2H₂O | 265 µg/ml |
| Glucose | 1.0 mg/ml | L-Glutamine | 292 µg/ml |
| L-Arginine-HCl | 126 µg/ml | L-Cystine-HCl -H₂O | 31.3 µg/ml |
| L-Histidine-HCl-H₂O | 42 µg/ml | L-Isoleucine | 52 µg/ml |
| L-Leucine | 52 µg/ml | L-Lysine-HCl | 72.5 µg/ml |
| L-Methionine | 15 µg/ml | L-Phenylalanine | 32 µg/ml |
| L-Threonine | 48 µg/ml | L-Tryptophan | 10 µg/ml |
| L-Tyrosine 2Na | 51.9 µg/ml | L-Valine | 46 µg/ml |
| Choline Chloride | 1.0 µg/ml | Folic Acid | 1.0 µg/ml |
| i-Inositol | 2.0 µg/ml | Niacinamide | 1.0 µg/ml |
| Pyridoxal HCl | 1.0 µg/ml | D-Pantothenic acid | 1.0 µg/ml |
| Riboflavin | 0.1 µg/ml | Thiamin HCl | 1.0 µg/ml |
| Phenol red Na | | | 11 µg/ml |

In some embodiments, the aforementioned maturation medium with respect to human oocytes may also include one or more antibiotics such as gentamicin, penicillin and streptomycin.

In some embodiments, the base medium is M199. In some embodiments, the base medium is supplemented with pyruvate, penicillin, streptomycin sulphate, L-glutamine, cysteamine, insulin, pregnant mare's serum gonadotropin (PMSG), human chorionic gonadotropin (HCG), epidermal growth factor (EGF) and follicular fluid. For example, this medium could be used for the *in vitro* maturation of a porcine oocyte given that most porcine IVM media include 10 to 20% follicular fluid. Follicular fluid may therefore be an optional constituent of the maturation medium when culturing oocytes from species other than pigs.

GM-CSF is a small secreted glycoprotein that folds into a bundled structure of four antiparallel α-helices. GM-CSF is produced by many cell types, including myeloid cells, dendritic cells (DCs), T cells, B cells, and non-haematopoietic cells (such as endothelial cells, fibroblast-like synoviocytes, chondrocytes, pulmonary epithelial cells). GM-CSF is also produced by cells of the reproductive tract (namely cells of the oviduct and endometrium).

The GM-CSF protein has a number of functions, primarily acting as a cytokine stimulating stem cells to produce granulocytes and monocytes. GM-CSF also plays a role in embryonic development, where it has been localized in the granulosa at protein and mRNA levels with concentrations being higher in the follicular fluid than in the serum. GM-CSF appears to be an essential local modulator implicated in the embryo implantation process given that neutralizing GM-CSF increases spontaneous abortion.

The implication of GM-CSF in embryo development and fetal survival was first described in mice. At an embryonic level, murine blastomere viability is enhanced by GM-CSF. It also promotes embryo development in the pig, sheep and cow, particularly by increasing IFNτ secretion in the latter two. GM-CSF has also been shown to have a positive trophic effect on human embryos. In this regard, the cellular mechanism implicated in this embryonic effect, by supplementing embryo culture media with human recombinant GM-CSF, is blastomere protection against apoptosis with no apparent effect on embryonic chromosomal constitution. However, a positive effect of GM-CSF on oocyte viability, maturity and developmental competence has not previously been reported.

To date, GM-CSF has been found in ninety three different mammalian species, including human, mouse, rat, cow, dog, pig, Rhesus monkey, sheep, chimpanzee, horse, cat, goat, fox, ferret, yak, macaque, cheetah, lemur, camel, bison, bear, opossum, dolphin, whale, tiger, seal, gorilla, elephant, panda, orangutan, rabbit, baboon and rhinoceros, amongst many others. GM-CSF is also referred to in the art as colony stimulating factor 2, CSF2, molgramostin and sargramostim.

GM-CSF for use in the present invention can be sourced a number of ways. For example, GM-CSF may be purchased commercially from available suppliers, including Sigma-Aldrich, ThermoFisher Scientific, Peprotech, Miltenyi Biotech, Kingfisher, Biotech, Inc., Bio-Rad, and others. In this regard, these suppliers provide GM-CSF in a purified form.

Typically, the GM-CSF is produced recombinantly in a non-human or non-animal host, such as *E. coli.* Techniques for recombinant production of proteins (including GM-CSF) and their purification are standard in the art. See, for example, Green MR and Sambrook J, Molecular Cloning: A Laboratory Manual (4th edition), Cold Spring Harbor Laboratory Press, 2012.

For recombinant production of GM-CSF, knowledge of the sequence of the GM-CSF gene in the desired species and encoded protein is required. In this regard, gene information may be accessed from the GenBank database at the National Center for Biotechnology Information (NCBI) (www.ncbi.nlm.nih.gov). For example, the Gene ID number for human GM-CSF is 1437. The human GM-CSF gene is represented by GenBank Accession Number NM_000758.3 (nucleotide sequence as represented by SEQ ID NO: 1) and NP_000749.2 (amino acid sequence as represented by SEQ ID NO: 2). Further details of the GM-CSF gene in other species may be accessed from the NCBI. For example, the Gene ID number for bovine GM-CSF is 281095, for pig is 397208, for horse is 100033981, for dog is 403923, for cat is 101094153, for mouse is 12981, and for sheep is 443400. The nucleotide and amino acid sequences of these species are represented by SEQ ID NOs: 3 to 16, respectively.

Details regarding the GM-CSF gene in humans and other species can also be found at the UniGene portal of the NCBI (UniGene Hs. 1349 - https://www.ncbi.nlm.nih.gov/UniGene/clust.cgi?UGID=130983&TAXID=9606&SEARCH=).

Alternatively, details of the nucleotide and amino acid sequence for GM-CSF can be accessed from the UniProt database (www.uniprot.org) wherein the UniProt ID for human GM-CSF is P04141.

It is to be made clear that reference herein to GM-CSF includes a reference to variants thereof. In this regard, a "variant" of GM-CSF may exhibit a nucleic acid or an amino acid sequence that is at least 80% identical, at least 90% identical, at least 95% identical, at least 98% identical, at least 99% identical, or at least 99.9% identical to native GM-CSF provided that the variant retains biological activity, or a substantial equivalent thereof, of the native GM-CSF. Such a variant of GM-CSF may be referred to as a "functionally active variant". Other levels of identity are contemplated, including variants with less than 80% identity.

A functionally active variant of GM-CSF may comprise individual amino acid substitutions, deletions or insertions relative to the native GM-CSF sequence (for example SEQ ID NO: 2 with respect to human GM-CSF). For example, a person skilled in the art will recognise that any amino acid can be substituted with a chemically (functionally) similar amino acid and retain function of the polypeptide. Such conservative amino acid substitutions are well known in the art. The following groups in Table 2 each contain amino acids that are conservative substitutions for one another.

**TABLE 2**

| **Exemplary amino acid conservative substitutions** | | | |
|---|---|---|---|
| **Original Residue** | **Exemplary Substitutions** | **Original Residue** | **Exemplary Substitutions** |
| Ala (A) | Val (V), Leu (L), Ile (I), Gly (G) | Leu (L) | Ile (I), Val (V), Met (M), Ala (A), Phe (F) |
| Arq (R) | Lys (K) | Lys (K) | Arq (R) |
| Asn (N) | Gln (Q), His (H) | Met (M) | Leu (L), Phe (F) |
| Asp (D) | Glu (E) | Phe (F) | Leu (L), Val (V), Ala (A) |
| Cys (C) | Ser (S) | Pro (P) | Gly (G) |
| Gln (Q) | Asn (N), His (H) | Ser (S) | Thr (T) |
| Glu (E) | Asp (D) | Thr (T) | Ser (S) |
| Gly (G) | Pro (P), Ala (A) | Trp (W) | Tyr (Y) |
| His (H) | Asn (N), Gln (Q) | Tyr (Y) | Trp (W), Phe (F) |
| Ile (I) | Leu (L), Val (V), Ala (A) | Val (V) | Ile (I), Leu (L), Met (M), Phe (F), Ala (A) |

Furthermore, if desired, unnatural amino acids or chemical amino acid analogues can be introduced as a substitution or addition into a polypeptide encompassed herein. Such amino acids include, but are not limited to, the D-isomers of the common amino acids, 2,4-diaminobutyric acid, α-amino isobutyric acid, 4-aminobutyric acid, 2-aminobutyric acid, 6-amino hexanoic acid, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogues in general.

When comparing amino acid sequences to define a variant of GM-CSF, the native and variant sequences should be compared over a comparison window which is determined by the length of the polypeptide. For example, a comparison window of at least 20 amino acid residues, at least 50 amino acid residues, at least 75 amino acid residues, at least 100 amino acid residues, or over the full length of the native GM-CSF is envisaged. The comparison window may comprise additions or deletions (i.e. gaps) of about 20% or less as compared to the reference sequence (which does not comprise additions or deletions) for optimal alignment of the two sequences. Optimal alignment of sequences for aligning a comparison window may be conducted by computerised implementations of algorithms such as the BLAST family of programs as, for example, disclosed by Altschul et al., 1997, Nucl. Acids Res. 25: 3389-3402. Global alignment programs may also be used to align similar sequences of roughly equal size. Examples of global alignment programs include NEEDLE (available at www.ebi.ac.uk/Tools/psa/emboss_needle/) which is part of the EMBOSS package (Rice P et al., 2000, Trends Genet., 16: 276-277), and the GGSEARCH program (available at fasta.bioch.virginia.edu/fasta_www2/fasta_www.cgi?rm=compare&pgm=gnw) which is part of the FASTA package (Pearson W and Lipman D, 1988, Proc. Natl. Acad. Sci. USA, 85: 2444-2448). Both of these programs are based on the Needleman-Wunsch algorithm which is used to find the optimum alignment (including gaps) of two sequences along their entire length. A detailed discussion of sequence analysis can also be found in Unit 19.3 of Ausubel et al ("Current Protocols in Molecular Biology" John Wiley & Sons Inc, 1994-1998, Chapter 15, 1998).

The amount of GM-CSF present in the medium is in the range of 0.1 ng/ml to 100 ng/ml. For example, the GM-CSF may be present in an amount from 0.1 to 90 ng/ml, 0.1 to 80 ng/ml, 0.1 to 70 ng/ml, 0.1 to 60 ng/ml, 0.1 to 50 ng/ml, 0.1 to 40 ng/ml, 0.1 to 30 ng/ml, 0.1 to 20 ng/ml, 0.1 to 10 ng/ml, 0.1 to 5.0 ng/ml, 0.1 to 4.0 ng/ml, 0.1 to 3.0 ng/ml, 0.1 to 2.0 ng/ml, 0.1 to 1.0 ng/ml, 0.1 to 0.5 ng/ml, 0.5 to 100 ng/ml, 0.5 to 90 ng/ml, 0.5 to 80 ng/ml, 0.5 to 70 ng/ml, 0.5 to 60 ng/ml, 0.5 to 50 ng/ml, 0.5 to 40 ng/ml, 0.5 to 30 ng/ml, 0.5 to 20 ng/ml, 0.5 to 10 ng/ml, 0.5 to 5.0 ng/ml, 0.5 to 4.0 ng/ml, 0.5 to 3.0 ng/ml, 0.5 to 2.0 ng/ml, 0.5 to 1.0 ng/ml, 1.0 to 100 ng/ml, 1.0 to 90 ng/ml, 1.0 to 80 ng/ml, 1.0 to 70 ng/ml, 1.0 to 60 ng/ml, 1.0 to 50 ng/ml, 1.0 to 40 ng/ml, 1.0 to 30 ng/ml, 1.0 to 20 ng/ml, 1.0 to 10 ng/ml, 1.0 to 9.0 ng/ml, 1.0 to 8.0 ng/ml, 1.0 to 7.0 ng/ml, 1.0 to 6.0 ng/ml, 1.0 to 5.0 ng/ml, 1.0 to 4.0 ng/ml, 1.0 to 3.0 ng/ml, and 1.0 to 2.0 ng/ml.

In some embodiments, the GM-CSF may be present in the medium in the range of 2.0 ng/ml to 10 ng/ml. For example, the GM-CSF may be present in an amount from 2.0 ng/ml to 9.0 ng/ml, 2.0 ng/ml to 8.0 ng/ml, 2.0 ng/ml to 7.0 ng/ml, 2.0 ng/ml to 6.0 ng/ml, 2.0 ng/ml to 5.0 ng/ml, 2.0 ng/ml to 4.0 ng/ml, 2.0 ng/ml to 3.0 ng/ml, 3.0 ng/ml to 10 ng/ml, 3.0 ng/ml to 9.0 ng/ml, 3.0 ng/ml to 8.0 ng/ml, 3.0 ng/ml to 7.0 ng/ml, 3.0 ng/ml to 6.0 ng/ml, 3.0 ng/ml to 5.0 ng/ml, 3.0 ng/ml to 4.0 ng/ml, 4.0 ng/ml to 10 ng/ml, 4.0 ng/ml to 9.0 ng/ml, 4.0 ng/ml to 8.0 ng/ml, 4.0 ng/ml to 7.0 ng/ml, 4.0 ng/ml to 6.0 ng/ml, 4.0 ng/ml to 5.0 ng/ml, 5.0 ng/ml to 10 ng/ml, 5.0 ng/ml to 9.0 ng/ml, 5.0 ng/ml to 8.0 ng/ml, 5.0 ng/ml to 7.0 ng/ml, 5.0 ng/ml to 6.0 ng/ml, 6.0 ng/ml to 10 ng/ml, 6.0 ng/ml to 9.0 ng/ml, 6.0 ng/ml to 8.0 ng/ml, 6.0 ng/ml to 7.0 ng/ml, 7.0 ng/ml to 10 ng/ml, 7.0 ng/ml to 9.0 ng/ml, 7.0 ng/ml to 8.0 ng/ml, 8.0 ng/ml to 10 ng/ml, 8.0 ng/ml to 9.0 ng/ml, and 9.0 ng/ml to 10 ng/ml.

In some embodiments, the amount of GM-CSF present in the medium may be about 2 ng/ml.

In some embodiments, the amount of GM-CSF present in the medium may be about 10 ng/ml.

The medium described hereinmay be used to mature an oocyte obtained from any mammalian origin. In some embodiments, the oocyte is selected from the group consisting of a human oocyte, a bovine oocyte, a porcine oocyte, an equine oocyte, a canine oocyte, a feline oocyte, a murine oocyte (e.g. mouse and rat), an ovine oocyte, and a non-human primate oocyte.

The subject from which the oocyte is obtained may be an otherwise healthy subject but for their reduced capacity or inability to conceive and/or carry a pregnancy to term. Alternatively, the subject may be aged and/or obese thereby also having a reduced capacity or inability to conceive and/or carry a pregnancy to term due to the effect of age and/or obesity on oocyte developmental competence. An aged subject would be considered a subject who is older than the peak fertility age range of the particular species. For an otherwise healthy human subject that range is between the ages of 23 and 35. Therefore an aged human subject could be considered a subject older than 35. Of course the peak fertility age range in humans may vary depending on a number of factors, including race, ethnicity, and genetic background.

The GM-CSF for inclusion in the medium is species-specific. That is, the source of the GM-CSF is the same as the source of the oocyte. For example, the GM-CSF may be recombinant or native human GM-CSF and the oocyte is a human oocyte. Alternatively, the GM-CSF may be recombinant or native porcine GM-CSF and the oocyte is a porcine oocyte.

Accordingly, in some embodiments, the oocyte is a porcine oocyte, the GM-CSF is porcine GM-CSF and the amount of porcine GM-CSF present in the medium may be about 2 ng/ml.

In some embodiments, the oocyte is a porcine oocyte, the GM-CSF is porcine GM-CSF and the amount of porcine GM-CSF present in the medium may be about 10 ng/ml.

In some embodiments, the oocyte is a bovine oocyte, the GM-CSF is bovine GM-CSF and the amount of bovine GM-CSF present in the medium may be about 2 ng/ml.

In some embodiments, the oocyte is a bovine oocyte, the GM-CSF is bovine GM-CSF and the amount of bovine GM-CSF present in the medium may be about 10 ng/ml.

In some embodiments, the oocyte is a murine oocyte, the GM-CSF is murine GM-CSF and the amount of murine GM-CSF present in the medium may be about 2 ng/ml.

In some embodiments, the oocyte is a murine oocyte, the GM-CSF is murine GM-CSF and the amount of murine GM-CSF present in the medium may be about 10 ng/ml.

In some embodiments, the oocyte is a human oocyte, the GM-CSF is human GM-CSF and the amount of human GM-CSF present in the medium may be about 2 ng/ml.

In some embodiments, the oocyte is a human oocyte, the GM-CSF is human GM-CSF and the amount of human GM-CSF present in the medium may be about 10 ng/ml.

Throughout the specification, reference to an oocyte encompasses an oocyte devoid of companion cells, or inclusive of companion cells. For example, the oocyte may be a denuded oocyte wherein the somatic cell layers (e.g. cumulus cells) that surround the oocyte have been removed. The oocyte may also be part of a follicle, or may be part of a cumulus oocyte complex (COC) in which the cumulus vestments remain intact.

As indicated above, it has been determined that maturation of oocytes harvested from the ovary is increased when the harvested oocyte is cultured in a medium comprising GM-CSF. In this regard, the success of assisted reproductive technologies depends to a large extent on the maturity of the oocyte prior to fertilization. Oocytes harvested from ovaries typically undergo spontaneous resumption of meiosis, i.e. proceed in their nuclear maturation, when placed in culture. This nuclear maturation may often occur before the oocyte has undergone other aspects of maturity, such as cytoplasmic maturity. An immature oocyte may ultimately affect the success of fertilization and possibly subsequent embryo implantation and development.

Indeed, the inventor has found that inclusion of GM-CSF in culture media for an oocyte, while increasing the maturity of the oocyte, also increases developmental competence of the oocyte. In this regard, the term "developmental competence" is to be understood to mean the capacity of the oocyte to develop into an embryo capable of implanting, developing to term and producing healthy offspring.

An "increase" in maturation and an "increase" in developmental competence is meant to refer to a level of maturity and developmental competence of the oocyte that is greater than that of an oocyte of the same species or from the same animal when cultured in a base medium which does not comprise GM-CSF. For example, the maturation and developmental competence of the oocyte cultured in a medium comprising GM-CSF may be increased by at least 5%, 10%, 20%, 30%, 40%, 50%, 60%, 70%, 80%, 90%, 100% or greater, 2-fold, 5-fold, 10-fold, 20-fold, 50-fold, or 100-fold or greater, relative to a control "untreated" oocyte.

As described herein, an increase in maturation and developmental competence may be assayed a number of ways. Ultimately, the most appropriate assays will evaluate positive effects on indicators of embryo viability and quality following fertilization of the "matured" oocyte. Suitable assays for example may examine for increased blastocyst inner cell mass number, increased blastocyst rate/number of blastocysts (measured for example by the number of oocytes that develop to the blastocyst stage after fertilization, and/or the number of oocytes that reach the blastocyst stage at the same time/rate as that would occur *in vivo* following normal/natural fertilization), increased trophectoderm cell number, increased total blastocyst cell number (the number of inner cell mass cells plus the number of trophectoderm cells), increased cleavage (measured for example by the ability of an oocyte to fertilize and divide to the two-cell stage), embryo morphology, number of blastocysts that hatch, pregnancy rate (measured for example by the success of pregnancy in a surrogate following transfer of an embryo derived from an oocyte cultured in a medium comprising GM-CSF, and/or by the number of horns which had implantations/fetuses present), and examination of DNA damage in a blastocyst or gene expression patterns. Alternatively, an appropriate assay will simply evaluate for an improvement in embryo viability following fertilization of the "matured" oocyte compared to known industry standards, or will test for the ability of the treated oocyte to develop into an embryo capable of implanting or developing to term (i.e. a birth), as indicated above. Other assays would be known to a person skilled in the art.

As described herein, with respect to an "increased blastocyst inner cell mass number", "increased blastocyst rate", "increased blastocyst trophectoderm cell number", "increased blastocyst total cell number", "increased cleavage", and "increased viability", the level of increase of each parameter compared to a relevant control (for example an embryo/blastocyst derived from an oocyte of the same species or from the same animal but which has not been exposed to GM-CSF, for example an oocyte cultured in a base medium which does not comprise GM-CSF) is taken to include at least a 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 90%, 100%, 105%, 110%, 120%, 130%, 140%, 150%, 160%, 170%, 180%, 190%, 200%, 210%, 220%, 230%, 240%, 250% or greater, 2-fold, 5-fold, 10-fold, 20-fold, 50-fold, or 100-fold or greater, increase.

Similarly, as described herein, an "increased pregnancy rate", "increased implantation" and "increased development to term" means an increase in the relevant parameter compared to a relevant control (for example an embryo/blastocyst which is derived from an oocyte of the same species or from the same animal but which has not been exposed to GM-CSF, for example an oocyte cultured in a base medium which does not comprise GM-CSF). That increase may be at least a 5%, 10%, 15%, 20%, 25%, 30%, 35%, 40%, 45%, 50%, 55%, 60%, 65% 70%, 75%, 80%, 85%, 90%, 95%, 100%, 105%, 110%, 120%, 130%, 140%, 150%, 160%, 170%, 180%, 190% or greater, 2-fold, 5-fold, 10-fold, 20-fold, 50-fold, or 100-fold or greater compared to the control.

Finally, as described herein, a "decrease" in DNA damage in a blastocyst means a level of damage that is less than that of a blastocyst derived from an oocyte of the same species or from the same animal when cultured in a base medium which does not comprise GM-CSF. A decrease in DNA damage is reflective of the ability of the oocyte maturation medium to reduce the environmental stress associated with *in vitro* culture of the oocyte. That decrease may be at least a 5%, 6%, 7%, 8%, 9%, 10%, 20%, 30%, 40%, 50%, 60%, 70%, 80%, 90%, 100%, 105%, 110%, 120%, 130%, 140%, 150%, 160%, 170%, 180%, 190% or less, 2-fold, 5-fold, 10-fold, 20-fold, 50-fold, or 100-fold or less, compared to the control.

As used herein, the term "derived from" includes an embryo which has been produced by *in vitro* fertilization of an oocyte. as described herein.

An oocyte can be harvested or collected from an ovary according to standard techniques long known in the art. For example, see Textbook of Assisted Reproduction: Laboratory and Clinical Perspectives (2003, *supra).* Most oocyte collection techniques involve the insertion of an aspirating needle into an ovarian follicle using transvaginal ultrasound. The aspirating needle is connected by tubing to a material collection trap and the collection trap, in turn, is connected to a suction source such as a manually operated syringe or an electromechanical vacuum source. Oocytes are typically isolated from multiple follicles. As such, harvested oocytes represent a heterogeneous population with regard to their maturity and therefore developmental potential.

As will be appreciated, the time for maturation of the oocyte in the medium of the present description may differ between species. Generally the time for maturation will be the time that the meiotic stage of metaphase II is reached in these systems, and as such the time for maturation will typically be from 6 hours to 44 hours for the oocyte to reach the metaphase II stage of meiosis. For example in the bovine setting the IVM time will generally be in the range from 18 - 24 hours in the absence of GM-CSF in the medium. In the human setting, the IVM time will generally be about 28 to about 52 hours, and most usually between about 30 to about 36 hours in the absence of GM-CSF in the medium.

In one embodiment, the oocyte is a porcine oocyte and the porcine oocyte is cultured in the medium of the present invention for about 40 to 42 hours at 35-39°C with a suitable gas mixture prior to fertilisation. An example of a suitable gas mixture includes, but is not limited to, a gas mixture comprising of CO₂ (1-10% by volume), balanced with air or with mixtures of O₂ and N₂ in proportions that sustain biological activity. For example, with respect to human oocytes incubation is typically at 37°C in an incubator with an atmosphere of 5% CO₂ and 95% air with high humidity (or with triple gas mixture (90% N₂, 5% CO₂, and 5% O₂) and 100% humidity).

In a further aspect, by establishing that inclusion of GM-CSF in a culture medium increases the maturation of an oocyte cultured in the medium *in vitro,* described herein is a method of *in vitro* maturation of an oocyte, the method comprising culturing the oocyte in a maturation medium as described above.

In a further aspect, described herein is a method for increasing maturation of an oocyte *in vitro,* the method comprising culturing the oocyte in a medium comprising GM-CSF. According to the present invention, the method for increasing maturation of an oocyte *in vitro* comprises culturing the oocyte *in vitro* in a mammalian oocycte maturation medium comprising 0.1 ng/ml to 100 ng/ml species-specific GM-CSF.

Given that presence of GM-CSF in the medium also increases developmental competence of the oocyte, in a further aspect described herein is a method of increasing developmental competence of an oocyte *in vitro,* the method comprising culturing the oocyte in a medium comprising GM-CSF.

As further described herein is an isolated oocyte with increased maturation and increased developmental competence produced by the aforementioned methods, and an embryo or non-human animal produced from the oocyte.

As described herein, the oocyte with increased maturation and increased developmental competence produced by the aforementioned methods *in vitro* may form part of an assisted reproductive technology. The term "assisted reproductive technology" as used throughout the specification is to be understood to mean any laboratory or clinical technology applied to isolated gametes (oocytes or sperm) and/or embryos for the purposes on reproduction. Such technologies include *in vitro* fertilization (IVF; aspiration of an oocyte, fertilization in the laboratory and transfer of the embryo into a recipient), gamete intrafallopian transfer (GIFT; placement of oocytes and sperm into the fallopian tube), zygote intrafallopian transfer (ZIFT; placement of fertilized oocytes into the fallopian tube), tubal embryo transfer (TET; the placement of cleaving embryos into the fallopian tube), peritoneal oocyte and sperm transfer (POST; the placement of oocytes and sperm into the pelvic cavity), intracytoplasmic sperm injection (ICSI), testicular sperm extraction (TESE), and microsurgical epididymal sperm aspiration (MESA); or any other *in vitro* technique for producing embryos in humans and/or animals, such as nuclear transfer, parthenogenic activation, embryonic stem cell production, and the use of totipotent cells.

As described herein, the assisted reproductive technology may comprise *in vitro* fertilization (IVF). IVF relates to the fertilization of an oocyte *in vitro,* wherein the oocyte is isolated from the subject and incubated in media to allow fertilization of the oocyte. As indicated above, methods are well known in the art for collecting oocytes from suitable females and fertilizing the oocytes *in vitro.* It is contemplated that fertilization of the oocyte will ideally occur no less than 24 hours, but no later than 60 hours, after collection of the oocyte and culturing in the medium described herein, such that maturity of the oocyte is at a sufficient stage to maximize the success of subsequent steps in the IVF procedure. For *in vitro* fertilization, the sperm may be incubated with the matured oocyte for a period of between 1 to 60 hours.

Accordingly, described herein is a method of producing an embryo from an oocyte by an assisted reproductive technology, the method comprising:
(a) collecting an oocyte from an ovary of a subject;
(b) culturing the oocyte *in vitro* in a medium comprising GM-CSF; and
(c) producing an embryo from the oocyte by fertilisation of the oocyte *in vitro.*

Described herein is a method of assisted reproduction involving an oocyte, the method comprising the step of exposing the oocyte to GM-CSF *in vitro.* In a still further aspect, there is described a method of assisted reproduction involving an oocyte, the method comprising the step of *in vitro* maturation of an oocyte by exposing the oocyte to GM-CSF *in vitro.* In some embodiments, the methods comprise the further step of exposing an embryo derived from the oocyte to GM-CSF *in vitro.*

As described herein, the assisted reproductive technology or the method of assisted reproduction is *in vitro* fertilisation.

In circumstances described herein where it is desired to accomplish fertilisation by other than natural interaction of sperm and oocyte, such as where the sperm is unable to fertilise the oocyte due to a thickened zona pellucida surrounding the oocyte, or where the sperm is from a male-factor patient, the sperm may be transported into the oocyte by a technique called intracytoplasmic sperm injection (ICSI). Accordingly, in some embodiments, the assisted reproductive technology or the method of assisted reproduction is ICSI. When the ICSI technique is used, the cumulus cells are removed from the oocyte, and sperm is injected into the interior of the oocyte using a glass pipette.

With respect to any of the aforementioned assisted reproductive technologies, as described herein the collected sperm may be maintained in a medium prior to fertilisation. A suitable medium would be known in the art and is set out in standard texts, such as the Textbook of Assisted Reproduction: Laboratory and Clinical Perspectives (2003, *supra*)*.* The medium containing the sperm may be of a constitution so as to minimise any stress placed on the oocyte when transferred from the maturation medium described hereinto the medium containing the sperm. Accordingly, in some embodiments the medium housing the sperm may have a similar or identical composition of ions and non-essential amino acids as the maturation medium.

The medium containing the sperm may comprise GM-CSF.

With respect to the fertilization process as described herein a suitable medium in which this is conducted (i.e. a fertilization medium) would be known in the art and is set out in standard texts, such as the Textbook of Assisted Reproduction: Laboratory and Clinical Perspectives (2003, *supra*)*.* The fertilization medium may be of a constitution so as to promote sperm function and fertilization. For example, the fertilization medium may comprise an elevated concentration of sodium and/or phosphate compared to the maturation medium described herein. The fertilization medium may also be supplemented with the carbohydrates glucose, lactate and pyruvate. Specific formulations may involve supplementation of the medium with one or more of bicarbonate, glutathione to promote sperm head decondensation, non-essential amino acids, HSA, hyaluronate, and antibiotics such as penicillin and streptomycin.

The fertilization medium may comprise GM-CSF.

Alternatively, as described herein, the collected sperm may be transferred directly into the maturation medium described herein which contains the matured oocyte (for *in vitro* fertilisation) or may be injected directly into a matured oocyte that is present in the maturation medium (for ICSI).

With respect to the ICSI technique as described herein, an alternative arrangement would be to use a single medium (an ICSI medium) that can be used to culture the matured oocyte and can also serve as a carrier for the sperm as it is transported by injection into the oocyte. The ICSI medium should preferably be highly compatible with the interior and exterior of the matured oocyte. The ICSI media may be a base medium as described above and may comprise ionic constituents similar to those found in the oocyte maturation medium described herein. In one embodiment, phosphate may be omitted to avoid metabolic and homeostatic stress on the matured oocyte. Because ICSI is a clinical procedure performed outside the incubator, a buffering system that is effective in a normal atmosphere is typically used. MOPS and HEPES are accordingly preferred buffers for the ICSI medium. Because the cumulus cells have been removed from the oocyte, and the sperm is at the conclusion of its independent life, glucose (the main energy source for cumulus cells and sperm, but not the oocyte) may be omitted from the ICSI medium. In order to nourish the matured oocyte, non-essential amino acids most abundant in the oocyte (e.g. glycine, proline, serine and taurine) and glutamine can be included in the ICSI medium to avoid osmotic and pH stress. The ICSI medium may also include hyaluronate or polyvinylpyrollidone (PVP) to immobilize or slow the sperm so that they may be captured in the ICSI pipette.

As described herein, the ICSI medium may comprise GM-CSF.

Following fertilisation as described herein, the embryo may be incubated in a medium which supports development of the embryo (an embryo culture medium). The embryo culture medium may be a base medium as described above and may comprise ionic constituents similar to those found in the oocyte maturation medium described herein. In one embodiment, the embryo culture medium may comprise EDTA which is believed to bind and disable toxins that may be deleterious to the early embryo. The embryo culture medium may also comprise HSA and hyaluronate. Furthermore, alanyl-glutamine may be substituted for glutamine to reduce ammonium build up within the medium.

The embryo culture medium may comprise GM-CSF.

As described herein, all media in which a harvested oocyte comes into contact, including from collection of the oocyte, to fertilization of the oocyte, and to subsequent embryo culture and development, may include GM-CSF. For example, it is expected that the presence of GM-CSF in the oocyte maturation media, the presence of GM-CSF in the medium containing the sperm, the presence of GM-CSF in the fertilization medium, and the presence of GM-CSF in media containing the oocyte following fertilization, will have an additive effect on blastocyst development, pregnancy rates, and ultimately development to term.

In some embodiments of the aforementioned methods, the amount of GM-CSF present in the respective media may be that as described above. For example, the GM-CSF may be present in the media in the range of 2.0 ng/ml to 10 ng/ml.

In further aspects, described herein is a granulocyte macrophage-colony stimulating factor (GM-CSF) for use, or when used, in a culture medium for increasing maturation of an oocyte *in vitro* or for increasing developmental competence of an oocyte *in vitro.* In another aspect, described herein is a use of a granulocyte macrophage-colony stimulating factor (GM-CSF) in the preparation of a culture medium for increasing maturation of an oocyte *in vitro* or for increasing developmental competence of an oocyte *in vitro.*

In further aspects, described herein is the preparation of a combination product for use in, or when used for, increasing maturation of an oocyte *in vitro* or for increasing developmental competence of an oocyte *in vitro.* In one aspect, a combination product may comprise:
(i) a culture medium;
(ii) granulocyte macrophage-colony stimulating factor (GM-CSF); and optionally
(iii) instructions for culturing an oocyte in the culture medium comprising the GM-CSF.

In another aspect, a combination product may comprise:
(i) a culture medium comprising granulocyte macrophage-colony stimulating factor (GM-CSF); and
(ii) instructions for culturing an oocyte in the culture medium.

A suitable culture medium for the combination product is described above with reference to the base medium. The source of GM-CSF has also been described above and in some embodiments is species-specific. The instructions may direct the user to the amount of GM-CSF to add to the medium, and may provide conditions which can be used for incubation of an oocyte in the medium. The instructions may be in the form of a suitable label or may be a separate insert.

The combination product may be used as part of an assisted reproductive technology. Examples of an assisted reproductive technology are described above.

It is to be noted that where a range of values is expressed, it will be clearly understood that this range encompasses the upper and lower limits of the range, and all values in between these limits.

The term "about" as used in the specification means approximately or nearly and in the context of a numerical value or range set forth herein is meant to encompass variations of +/- 10% or less, +/- 5% or less, +/- 1% or less, or +/- 0.1% or less of and from the numerical value or range recited or claimed.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

As used herein, the singular forms "a," "an," and "the" may refer to plural articles unless specifically stated otherwise.

All methods described herein can be performed in any suitable order unless indicated otherwise herein or clearly contradicted by context. The use of any and all examples, or exemplary language (e.g., "such as") provided herein, is intended merely to better illuminate the example embodiments and does not pose a limitation on the scope of the claimed invention unless otherwise claimed. No language in the specification should be construed as indicating any non-claimed element as essential.

Furthermore, the description provided herein is in relation to several embodiments which may share common characteristics and features. It is to be understood that one or more features of one embodiment may be combinable with one or more features of the other embodiments. In addition, a single feature or combination of features of the embodiments may constitute additional embodiments.

The invention is further illustrated in the following examples. The examples are for the purpose of describing particular embodiments only and are not intended to be limiting with respect to the above description. It will be appreciated by those skilled in the art that the disclosure may be embodied in many other forms.

### EXAMPLE 1

### Effect of GM-CSF on Maturation of Porcine Oocytes in vitro

The effect of adding granulocyte macrophage-colony stimulating factor (GM-CSF) to various oocyte maturation media was examined in pigs in dose response experiments using recombinant GM-CSF. Embryos were produced using *in vitro* embryo production systems. Experiments examined the number of embryos that cleaved and developed to the blastocyst stage as well as blastocyst inner cell mass, trophectoderm and total cell numbers as measures of oocyte developmental competence. All chemicals were purchased from Sigma-Aldrich unless otherwise stated.

### METHODS

### 1. Oocyte Maturation and In vitro Embryo Production - Method 1

### 1.1 Oocyte Collection and in vitro Maturation

Porcine ovaries were collected from a local abattoir and transported to the laboratory in a 0.9% sodium chloride solution at between 33°C and 37°C. Small antral follicles between 3 to 6 mm in diameter were aspirated using a 21-gauge needle connected to a constant vacuum source. The follicular contents were pooled in collection tubes to be searched through using a dissection microscope.

Cumulus-oocyte complexes (COCs) with at least three compact cumulus layers and an evenly granulated cytoplasm were recovered from the collected fluid and pooled in a 1 ml drop of Medium 199 Hepes (M199 HEPES; Life Technologies, Vic, AUS - now ThermoFisher Scientific) with 10% sow follicular fluid under oil. Once all the collection tubes were processed the pooled COCs were washed three times through a basic maturation media. During the last wash the COCs went through a final selection process were any unsuitable COCs were removed. The remaining COCs were allocated randomly into groups of 50-60 COCs then cultured in basic maturation media supplemented with 0 ng/ml, 2 ng/ml or 10 ng/ml of porcine GM-CSF. Each treatment group was cultured in 600 µl of maturation media covered with mineral oil in the well of a 4-well nunclon IVF multidish for 40-42 hrs in an atmosphere of 5% CO₂ in air at 38.5°C.

The basic maturation media consisted of 50 ml of Medium 199 (M199; Life Technologies, Vic, AUS - now ThermoFisher Scientific) supplemented with 5.0 mg Na-pyruvate, 3.8 mg penicillin-G, 2.5 mg streptomycin sulphate, 5 mg L-glutamine, 5 µL cysteamine (5M stock), and the maturation working solution contained 9 ml of the basic media supplemented with 10 µl insulin (5 mg/ml stock), 100 µl PMSG (1000 iu/ml stock), 100 µl HCG (1000 iu/ml stock), 5 µl EGF (10 µg/ml stock) and 1 ml filtered sterilized sow follicular fluid. Follicular fluid was thawed and filter sterilized (Millipore, USA; filter pore size 0.22µm) immediately prior to use.

### 1.2 In vitro Fertilization (IVF) and in vitro Culture (IVC)

After maturation, the COCs were co-incubated with mixed boar semen (Landrace/Large White) for 6 h in an atmosphere of 5% CO₂ in air at 38.5°C. Fertilization was performed in 100 µl droplets of TALP-PVA fertilization medium under mineral oil (Bavister BD, 1989, A consistently successful procedure for in vitro fertilization of golden hamster eggs, Gamete Research, 23(2): 139-158). TALP-PVA stock medium contained 114.0 mM NaCl, 3.16 mM KCI, 4.72 mM CaCl₂•2H₂O, 0.5 mM MgCl₂•6H₂O, 25 mM NaHCO₃, 5 mM D-Glucose, 0.075 mg/ml penicillin-G, 0.05 mg/ml streptomycin sulphate, and 1.0 mg/ml PVA and the working solution was supplemented with 10 mM Na-lactate, 0.1 mM Na-pyruvate, 2 mM caffeine-sodium benzoate, 3 mM Ca-lactate2pentahydrate and 3 mg/ml of BSA (fraction V). Sperm was prepared by centrifuging the semen twice at 1300 RPM for 5 min and washed with Sperm Pre-incubation Media composed of M199 supplemented with 5 mg Na-pyruvate, 0.9 mg/ml Ca-lactate, 0.075 mg/ml penicillin, 0.05 mg/ml streptomycin sulphate, 6 ml heat inactivated FBS and 0.1 mg/ml of L-glutamine, before diluting with TALP-PVA medium to give a final sperm concentration of 5x10⁶ sperm/ml. 10 µl of the prepared sperm was added to 90 µl droplets of TALP-PVA medium containing the matured COCs to give a final sperm concentration of 5x10⁵ sp/ml.

Following fertilization, the cumulus cells were removed by repetitive pipetting and the zygotes washed twice through modified NCSU-23 (NCSU-PLG) supplemented with 0.2 mM pyruvate, 5.7 mM Na-lactate, 0.6 mM glucose & MEM-nonessential amino acids (Invitrogen, USA) and then cultured in 50 µl NUSU-PLG drops under mineral oil for day's 1 to 3. On the 3rd day embryos were checked for cleavage and transferred into new drops of modified NCSU-23(NCSU-G), supplemented with 5.5 mM glucose and MEM non-essential and essential amino acids (Invitrogen, USA) for day's 3 to 6. On the 5th day post fertilization, 10% foetal bovine serum was added to the culture drops and morphology assessed.

Embryos were cultured for 6 days in a humidified atmosphere of 5% CO₂, 5% O₂ and the balance Nitrogen at 38.5°C. Blastocyst development was determined on Day 5 and Day 6. Day 6 blastocysts were differentially stained to determine total cell number, inner cell mass number and trophectoderm cell number.

On Day 6 of embryo development blastocysts were scored based upon morphological appearance. Blastocysts were scored as being either; small, medium, expanded or hatched/hatching based upon blastocyst diameter and cavity size.

### 2. Oocyte Maturation and In vitro Embryo Production - Method 2

### 2.1 Oocyte Collection and in vitro Maturation

Ovaries were collected from a local abattoir and transported for 2 hrs at 37°C to the laboratory in a 0.9% sodium chloride saline solution. In the laboratory, small antral follicles (3-6 mm in diameter) were aspirated using an 18-20 guage syringe.

Cumulus-oocyte complexes (COCs) were removed and selected based upon morphological appearance. COCs with fewer than three layers of cumulus cells, with an overly expanded and uneven or dark cumulus layer, or with abnormal nuclei, were excluded. COCs were washed once through Medium 199 HEPES (M199 HEPES; Life Technologies, Vic, AUS - now ThermoFisher Scientific) and then twice through BOMED maturation media (Kühholzer B et al., 2001, Clonal lines of transgenic fibroblast cells derived from the same fetus result in different development when used for nuclear transfer in pigs, Biology of Reproduction, 64(6): 1695-1698) supplemented with 3 mg/ml polyvinyl alcohol (PVA). The basic maturation media consisted of 50 ml of Medium 199 (Invitrogen, Carlsbad, CA, USA) supplemented with 5.0 mg Na-pyruvate, 3.8 mg penicillin-G, 2.5 mg streptomycin sulphate, and 5 µl cysteamine (5 M stock). The final or working BOMED maturation media contained 10 ml of the basic maturation media supplemented with 10 µl insulin (5 mg/ml stock), 100 µl PMSG (1000 iu/ml stock, Folligon, Intervet, Castle Hill, Australia), 100 µl HCG (1000 iu/ml stock, Chorulon, Intervet, Castle Hill, Australia), 5 µl EGF (10 µg/ml stock) and 3 mg/ml PVA (Beebe LFS et al., 2007, The effect of energy substrate concentration and amino acids on the in vitro development of preimplantation porcine embryos, Cloning and Stem Cells, 9(2): 206-215). For each treatment group (0 ng/ml, 2 ng/ml or 10 ng/ml of porcine GM-CSF), 50 COCs were cultured in 600 µl of maturation media covered with mineral oil in the well of a 4-well nunclon IVF multidish for 40-42 hrs in an atmosphere of 5% CO₂ in air at 38.5°C.

### 2.2 In vitro Fertilization (IVF) and in vitro Culture (IVC)

After maturation, the COCs were co-incubated with mixed boar semen (Landrace/Large White; SABOR Pty Ltd Clare SA) for 6 hrs at 38.5°C in 5% CO₂ air. Fertilization was performed in 100 µl droplets of TALP-fertilization medium (Bavister BD, 1989 *supra*) under mineral oil. TALP-fertilization medium contained 25 ml of TALP-PVA stock solution (114 mM NaCl, 3.16 mM KCI, 4.72 mM CaCl₂•2H₂O, 0.5 mM MgCl₂•6H₂O, 25 mM NaHCO₃, 5 mM D-Glucose, 0.0188g/250ml penicillin-G, 0.0125g/250ml streptomycin sulfate, 0.0005g/250ml phenol red and 0.25g/250ml PVA) supplemented with 10 mM Na-lactate, 0.1 mM Na-pyruvate, 2 mM caffeine-sodium benzoate, 3 mM Ca-lactate2pentahydrate and 75 mg of BSA (fraction V). Sperm was prepared by centrifuging the semen twice at 1400 RPM for 5 min before diluting with TALP-fertilization medium and sperm wash (composed of 50 ml M199 supplemented with 5 mg Na-pyruvate, 45 mg Ca-lactate, 3.75 mg penicillin-G, 2.5 mg streptomycin sulfate and 6 ml heat-inactivated FBS) to give a final sperm concentration of 5x10⁶ sperm/ml. 10 µl of the prepared sperm was added to 90 µl droplets of TALP-fertilization medium containing the matured COCs, to give a final sperm concentration of 5x10⁵ sperm/ml.

Following fertilization, the cumulus cells were removed by repetitive pipetting and the zygotes washed twice through modified PZM-5 (mPZM-5; Yoshioka K et al., 2008, Defined system for in vitro production of porcine embryos using a single basic medium, Journal Reproduction and Development, 54(3): 208-213) and then cultured in 100 µl mPZM-5 IVC droplets. Modified PZM-5 culture medium was supplemented with 3 mg/ml of BSA instead of PVA (Yoshioka K et al., 2012, Production of piglets from in vitro-produced embryos following non-surgical transfer, Animal Reproduction Science, 131(1-2): 23-29). Embryos were cultured for 6 days at 38.3°C in 5.5% CO₂ and 7% O₂ humidified air. Cleavage was determined on Day 3 and blastocyst development rate was determined on Day 5 and Day 6. Blastocyst development rate was scored based upon morphological appearance, including blastocyst diameter and cavity size. Blastocysts were scored as being either; small-medium, expanded or hatched/hatching.

### Differential Staining

For both embryo production methods described above, blastocysts scored as medium, expanded, or hatched/hatching, were differentially stained on Day 6 of culture with Hoechst 33358 and Propidium Iodide (Handyside A and Hunter S., 1984, Rapid procedure for visualising the inner cell mass and trophectoderm nuclei of mouse blastocysts in situ using polynucleotide-specific fluorochromes, J. Exp. Zool., 231: 429-434). The zona pellucida of each embryo was removed in a 200 µl drop of pronase solution under mineral oil and the zona free blastocysts were washed twice in protein free M199 Hepes containing 1 mg/ml PVA. The blastocysts were then incubated in the dark in 60 µl of 10 mM trinitrobenzenesulphonic acid (TNBS) with 740 µl M199 Hepes-PVA, at a pH of 8.5 for 20 min. The blastocysts were then washed three times through M199 Hepes-PVA and then incubated in 10 µl of 0.2 mg/ml anti-DNP BSA with 40 µl M199 Hepes-PVA for 20 min at 37°C. The blastocysts were then washed three times through M199 Hepes-PVA before incubation in 5 µl guinea pig complement with 10 µg/ml Hoechst 33342 (1 mg/ml stock), 10 µg/ml Propidium iodide (0.1 mg/ml stock) and 40 µl M199 Hepes-PVA for 20 mins at 37°C. Following a final wash through M199 Hepes-PVA, the blastocysts were fixed in 99% ethanol and then mounted on a glass slide under glycerol and covered with a cover slip. The cover slip was then sealed with nail polish. The stained embryos were viewed using a UV light source microscope. The inner cell mass stained blue and the trophectoderm cells stained pink.

### Porcine GM-CSF

Porcine recombinant GM-CSF was purchased from R&D systems (Minneapolis, Minnesota). Lyophilized 10 µg of GM-CSF (product 711-PG-010) was reconstituted to 10 µg/ml in sterile D-PBS containing 0.1% BSA. This solution was further diluted 1:10 in sterile D-PBS containing 0.1% BSA to give a 1 µg/ml (1000x) stock solution and stored at -20⁰C until use. This stock solution was then diluted either 1:500 or 1:100 to give a working concentration of either 2 ng/ml or 10 ng/ml.

### RESULTS

### The addition of GM-CSF to oocyte maturation media increases blastocyst inner cell mass cell numbers in pigs

In the first oocyte maturation and embryo production method, abattoir-derived porcine oocytes were matured in oocyte maturation medium containing 10% porcine follicular fluid with or without supplemented GM-CSF. The effect of GM-CSF on blastocyst inner cell mass number, trophectoderm cell number, and total cell number is shown in Table 3. Treatment with 2 ng/ml GM-CSF significantly increased blastocyst inner cell mass, trophectoderm and total cell numbers by 60.8%, 8.6%, and 12.7%, respectively, compared to the control group.

**TABLE 3**

| Effect of GM-CSF on blastocyst ICM, trophectoderm and total cell number | | | | |
|---|---|---|---|---|
| **GM-CSF (ng/ml)** | **n** | **ICM** | **Trophectoderm** | **Total** |
| 0 | 64 | 3.39 ± 0.62 | 39.91 ± 3.24 | 43.30 + 3.64 |
| 2 | 70 | 5.45 ± 0.52 | 43.33 ± 2.31 | 48.78 ± 2.65 |
| 10 | 76 | 5.35 ± 0.43 | 43.73 ± 2.54 | 48.76 ± 3.01 |

| | | | | |
|---|---|---|---|---|
| Values are mean ± standard error (SEM) of 5 replicates. | | | | |

### The addition of GM-CSF to oocyte maturation media increases on-time blastocyst development and blastocyst rate in pigs

In the second oocyte maturation and embryo production method, abattoir-derived porcine oocytes were matured in oocyte maturation medium with or without supplemented GM-CSF and lacking porcine follicular fluid. The effect of GM-CSF on subsequent embryo development is shown in Table 4. Treatment with 2 ng/ml and 10 ng/ ml of GM-CSF significantly increased the number of blastocysts present on day 5 of culture by 41% and 78%, respectively. This is the stage that blastocysts would normally form *in vivo* demonstrating that the addition of GM-CSF to maturation media can overcome the developmental delay seen when embryos are produced and/or cultured *in vitro* to increase on-time blastocyst development. The addition of GM-CSF to oocyte maturation media also increased the number of blastocysts present at the end of culture on day 6 by 28% and 22%, respectively.

**TABLE 4**

| **Effect of GM-CSF on porcine embryo development** | | | | |
|---|---|---|---|---|
| **GM-CSF (ng/ml)** | **n** | **Cleaved** | **Day 5 Blastocyst** | **Day 6 Blastocyst** |
| 0 | 289 | 65.0 (3.8) | 13.8 (2.1) | 29.5 (4.3) |
| 2 | 275 | 79.8 (3.9) | 19.4 (4.1) | 38.0 (4.3) |
| 10 | 279 | 73.1 (4.9) | 24.6 (3.6) | 36.1 (4.5) |

| | | | | |
|---|---|---|---|---|
| Values are percentage of total (n) and are mean ± standard error (SEM) of 6 replicates | | | | |

The blastocysts above were differentially stained to determine inner cell mass, trophectoderm and total cell numbers. The results are shown in Table 5. The addition of 2 ng/ml of GM-CSF to the ooycte maturation media increased blastocyst inner cell mass, trophectoderm and total cell numbers by 25%, 11% and 13%, respectively. The addition of 10 ng/ml of GM-CSF to the oocyte maturation media increased blastocyst inner cell mass, trophectoderm and total cell numbers by 29%, 16% and 19%, respectively compared with the control group.

**TABLE 5**

| **Effect of GM-CSF on blastocyst ICM, trophectoderm and total cell number** | | | | |
|---|---|---|---|---|
| **GM-CSF (ng/ml)** | **n** | **ICM** | **Trophectoderm** | **Total** |
| 0 | 81 | 7.38 (0.9) | 38.3 (2.3) | 45.7( 2.5) |
| 2 | 101 | 9.25 (1.0) | 42.5 (3.0) | 51.6 (4.0) |
| 10 | 98 | 9.55 (1.5) | 44.3 (2.9) | 54.5 (4.6) |

| | | | | |
|---|---|---|---|---|
| Values are mean ± standard error (SEM) of 6 replicates. | | | | |

### EXAMPLE 2

### Effect of GM-CSF on Maturation of Bovine Oocytes in vitro

The effect of adding granulocyte macrophage-colony stimulating factor (GM-CSF) to oocyte maturation media was examined in cows in dose response experiments using recombinant GM-CSF. Embryos were produced using *in vitro* embryo production systems. Experiments examined the number of embryos that cleaved and developed to the blastocyst stage as well as blastocyst inner cell mass, trophectoderm and total cell numbers as measures of oocyte developmental competence. All chemicals were purchased from Sigma-Aldrich unless otherwise stated.

### METHODS

### Bovine GM-CSF

Bovine recombinant GM-CSF was purchased from Kingfisher Biotech, Inc. (St Paul, Minnesota). Lyophilized 25 µg of GM-CSF (product RP0871B-025) was reconstituted to 25 µg/ml in sterile D-PBS containing 0.1% BSA. This solution was further diluted 1:10 in sterile D-PBS containing 0.1% BSA to give a 1 µg/ml (1000x) stock solution and stored at -20⁰C until use. This stock solution was then diluted either 1:500 or 1:100 to give a working concentration of either 2 ng/ml or 10 ng/ml.

### Oocyte Maturation and In vitro Embryo Production

### Oocyte Collection and in vitro Maturation

Ovaries were collected and transported from a local abattoir in saline at 30-35°C. Follicles between 2 and 8 mm in diameter were aspirated using an 18-gauge needle and 10 ml syringe. COCs with tight cumulus and ungranulated oocytes were then selected in clean follicular fluid, washed twice through wash media (Vitrowash, IVF Vet Solutions) + 4.0 mg/ml BSA, and *in vitro* maturation (IVM) media (Vitromat, IVF Vet Solutions, Robinson Research Institute, University of Adelaide, SA, Australia) supplemented with 4.0 mg/ml BSA and 0.1 IU/ml FSH (Ovagen). The COCs were then transferred into equilibrated IVM medium culture drops (500 µl drops) covered with paraffin oil (Merck). Maturation treatment groups were: (1) control (IVM medium), (2) 2ng/ml GM-CSF (IVM medium + 2 ng/ml GM-CSF), and (3) 10 ng/ml GM-CSF (IVM medium + 10 ng/ml GM-CSF). Groups of 50 COCs were cultured in 500 µl media for 23 hrs at 38.5°C with 6% CO₂ in humidified air.

### In vitro Fertilization (IVF) and in vitro Culture (IVC)

After maturation, the COCs were washed through wash medium (Vitrowash, IVF Vet Solutions) + 4.0 mg/ml BSA, and transferred into 500 µl culture drops of IVF medium (Vitrofert, IVF Vet Solutions) + 4.0 mg/ml BSA + 10 IU Heparin + 25 µM penicillamine + 12.5 µM hypotaurine + 1.25 µM epinephrine covered with paraffin oil. Sperm was supplied by Semex Australia Pty Ltd from a fertility proven bull. Two straws of sperm were thawed at 30-35°C and prepared using a Bovipure discontinuous gradient (40%:80%) and added at a final concentration of 1 X 10⁶ sperm/ml. COCs were incubated with sperm for 23 hrs in 6% CO₂ in air before being mechanically denuded by repeat pipetting and washed through wash media (Vitrowash, IVF Vet Solutions) + 4.0 mg/ml BSA, and groups of 5 embryos were transferred into 20 µl drops of pre-equilibrated cleavage medium (Vitrocleave, IVF Vet Solutions) + 4.0 mg/ml BSA covered with paraffin oil and incubated at 38.5°C in 7% O₂, 6% CO₂ and balance of N₂ for 5 days. On day 5, embryos were transferred to 20 µl culture drops of pre-equilibrated blastocyst medium (Vitroblast, IVF Vet Solutions) + 4.0 mg/ml BSA covered with paraffin oil at 38.5°C in 7% O₂, 6% Co₂ and balance of N₂ until day 8.

### RESULTS

### The addition of GM-CSF to oocyte maturation media increases blastocyst development in cattle

Abattoir-derived bovine oocytes were matured in oocyte maturation media with or without supplemented GM-CSF. The effect of GM-CSF on subsequent embryo development is shown in Table 6. The addition of 2 ng/ml and 10 ng/ml of GM-CSF increased the number of blastocysts present on day 8 of culture by 20% and 44%, respectively, compared with the control group.

**TABLE 6**

| **Effect of GM-CSF on bovine embryo development** | | | |
|---|---|---|---|
| **GM-CSF (ng/ml)** | **n** | **Cleaved** | **Day 8 Blastocyst** |
| 0 | 179 | 92 (3.5) | 36.9 (5.9) |
| 2 | 173 | 88 (6.1) | 44.1 (5.6) |
| 10 | 160 | 89 (5.7) | 53.2 (5.6) |

| | | | |
|---|---|---|---|
| Values are percentage of total (n) and are mean ± standard error (SEM) of 4 replicates | | | |

### The addition of GMCSF to oocyte maturation media increases the proportion of bovine blastocysts that are hatched blastocysts on Day 8 of embryo culture

As shown in Figure 1, treatment with 2 ng/ml and 10 ng/ ml of GM-CSF significantly increased the proportion of total bovine blastocysts that reached the hatched blastocyst stage on day 8 of embryo culture by 45.4% and 149.0%, respectively. This is the stage that blastocysts would normally form *in vivo* demonstrating that the addition of GM-CSF to maturation media can overcome the developmental delay seen when bovine embryos are produced and/or cultured *in vitro* to increase on-time blastocyst development.

Demonstration of an efficacy for the use of GM-CSF in maturing bovine oocytes *in vitro* is important given that the beef and dairy cattle industries already use IVM routinely for breeding purposes, and cattle embryos are often used as a model for effects on human embryos.

### EXAMPLE 3

### Effect of GM-CSF on Maturation of Murine Oocytes in vitro

The effect of adding granulocyte macrophage-colony stimulating factor (GM-CSF) to oocyte maturation media was examined in mice in dose response experiments using recombinant GM-CSF. Embryos were produced using *in vitro* embryo production systems. Experiments examined the number of embryos that cleaved and developed to the blastocyst stage as well as blastocyst inner cell mass, trophectoderm and total cell numbers as measures of oocyte developmental competence. All chemicals were purchased from Sigma-Aldrich unless otherwise stated.

### Mice

CBAFI males (6-8 weeks) and females (21-23 days) were housed under a 12 h light and 12 h dark cycle with *ad libitum* access to water and food. All experiments were performed in accordance with Australian Code of Practice for the care and the Use of Animals for Scientific Purpose and the study was approved by the University of Adelaide Animal Ethics Committee (M/2015/072 and M/2017/081).

### Murine GM-CSF

Murine recombinant GM-CSF was purchased from R&D systems (Minneapolis, Minnesota). Lyophilized 10 µg of GM-CSF (product 415-ML-010) was reconstituted to 10 µg/ml in sterile D-PBS containing 0.1% BSA. This solution was further diluted 1:10 in sterile D-PBS containing 0.1% BSA to give a 1 µg/ml (1000x) stock solution and stored at -20⁰C until use. This stock solution was then diluted either 1:500 or 1:100 to give a working concentration of either 2 ng/ml or 10 ng/ml.

### Oocyte Maturation and In vitro Embryo Production

### Oocyte Collection and in vitro Maturation

C57BI6 x CBA F1 female mice were administered with equine chorionic gonadotropin (eCG; Folligon, Intervert, Boxmeer, The Netherlands) via intraperitoneal injection (i.p) to stimulate follicle growth. 46-48 h post-injection, cumulus oocyte complexes (COCs) were aspirated from large antral follicles and collected in HEPES-buffered minimum essential medium (α-MEM) handling media supplemented with 4.0 mg/ml bovine serum albumin (BSA) and 1 mg/ml Fetuin (Wong SL et al., 2015, Hyperglycaemia and lipid differentially impair mouse oocyte developmental competence, Reproduction, Fertility and Development, 27: 583-592). IVM Media was pre-equilibrated in an incubator (37°C, 6% O₂) for 4 h before performing IVM. Ten COCs were cultured per 50 µl drop of IVM media in 20% O₂, 6% CO₂, and N₂ as balance for 16 h. The IVM culture medium was bicarbonate-buffered α-MEM containing 3 mg/ml BSA and 1 mg/ml Fetuin and 5 mIU/ml FSH (Puregon-Organon, Oss, The Netherlands; Wong SL *et al.,* 2015, *supra*) 16 h post-maturation, cumulus expansion was assessed using a scale as described in Vanderhyden BC et al., 1990 (Developmental pattern of the secretion of cumulus expansion-enabling factor by mouse oocytes and the role of oocytes in promoting granulosa cell differentiation, Developmental Biology, 140: 307-317). COCs were graded as 0: no expansion, 1+: outer layer of cumulus cells expanded, 2: outer half of cumulus expanded, 3: all layers expanded apart from corona radiatae, and 4+: maximum expansion of all layers of cumulus cells.

### In vitro Fertilization (IVF) and in vitro Culture (IVC)

C57BI6 x CBA F1 male mice were sacrificed using cervical dislocation and the Vas deferens along with the epididymis dissected out and placed in Wash Media (Cook Medical Pty. Ltd, QLD, Australia) at 37°C, and excess tissue and fat removed under microscope. The vas deferens and epididymis were then transferred into a culture dish containing pre-equilibrated 1000 µl Fert Media (Cook Medical Pty. Ltd, QLD, Australia) and spermatozoa extracted and incubated for 45-60 min at 37°C in 6% CO₂, 5% O₂, and 89% N₂ to allow the sperm to capacitate (Wong SL *et al.,* 2015, *supra*) Spermatozoa, were then added to the fertilization drop containing expanded COCs and incubated for 4 h. Following fertilization presumptive zygotes were cultured in Cleave Media (Cook Medical Pty Ltd, QLD, Australia) until day 5. Preimplantation embryo development was determined by examining cleavage rate as a measure of fertilization and blastocyst rates on days 4 and 5.

### Differential Staining

ICM and trophectoderm (TE) cell numbers were determined using differential staining as described (Handyside A and Hunter S., 1984 *supra*)*.* Blastocysts were placed in 20 µl of 0.5% pronase for about 2-3 min until the zona pellucida was dissolved and then placed in protein free 3-(N-morpholino) propanesulfonic acid (MOPS) media. These were then transferred into 10 µl of 2,4,6-trinitrobenzenesulfonic acid and 90 µl of plolyvinylpyrrolidone (PVP) and cultured at 4°C for 10 min. Blastocysts were then incubated in 20 µl of anti-dinitrophenyl for 10 minutes at 37°C and then incubated in complement (50 µl propidium iodide (PI) and 50µl guinea pig serum) at 37°C for 5 min. Blastocysts were then transferred to 500 µl of bisbenzimide and incubated overnight at 4°C. The following day embryos were placed in 500 µl of 100% ethanol and placed on a siliconised slide in 3 µl of glycerol. A coverslip was gently placed on top of the drop and the number of ICM cells (blue) and TE (pink) counted under UV and red filter. Blastocysts were imaged using Olympus BX 51 (Olympus, Victoria, Australia) fitted with an ultraviolet lamp. Bisbenzimide was excited and emitted at 338 nm and 505 nm to visualise ICM cells and PI at 537 nm and 619 nm to visualise TE cells.

### Blastocyst DNA Damage

The histone modification antibody γH2AX (Cell Signalling Technology) was used to measure the incidence of DNA double strand breaks as an estimate of blastocyst DNA damage (Sharma A et al., 2012, Histone H2AX phosphorylation: A marker for DNA damage, Methods in Molecular Biology, 920: 613-626). Embryos were fixed in 4% paraformaldehyde and then washed for 30 min in phosphate buffered saline (PBS) with 0.3 mg/ml polyvinyl alcohol (PVA) and permeabilised in 0.25% Triton-X (USB Corporation, OH). To prevent non-specific binding, 10% blocking solution of goat serum in PBS-PVA (Jackson ImmunoResearch, PA) was added for 1 h. Blastocysts were then incubated overnight at room temperature (RT) with primary antibody γH2AX in 10% goat serum (in PBS-PVA). No primary antibody was used in the negative control. On Day 2, blastocysts in treatments were washed in PBS-PVA solution for 3 times for 2 min each. Blastocysts were then labelled with secondary antibody, Alexa-594 (Life Technologies) at a dilution factor of 1:500 and incubated for 2 h at room temperature. Along with secondary antibody, 4'6 dimidino-2-phenylindole (DAPI) was added as a nuclear stain. Post-incubation embryos were washed three times in PBS and were loaded into a confocal dish for imaging. Fluorescence was detected by a Fluoview FV10i confocal microscope (Olympus, Tokyo, Japan). Instrument settings were kept constant for each replicate. The experiment was replicated five times with at least 10 blastocysts evaluated per treatment.

### Vitrification/Warming

Mouse blastocyst were collected on day 5 and vitrified prior to embryo transfer to pseudopregnant recipient mice. Vitrification mediums were made on the day vitrification was performed. For vitrification, the handling media (HM) contained BSA, the equilibrium solution consisted of 8 ml handling media, 1 ml ethylene glycol (EG), 1 ml dimethyl sulphoxide (DMSO), and the vitrification solution consisted of 10.8 ml sucrose media (SM), 1.2 ml handling media, 3 ml DMSO and 3 ml ethylene glycol. 600 µl was taken from each media and placed in a 4-well nunc dish which was equilibrated at 37°C for 15 min before performing vitrification. 6-7 blastocyst were transferred into each well containing handling media and then immediately placed in another well containing handling media, then into well 3 with equilibrium solution for 3 min at 37°C and then into vitrification solution for 20-30 sec at 37°C before placing it onto the hook of the straw which was then inserted into precooled fibre plug and stored in liquid nitrogen. Warming media was made on the morning of embryo transfer and equilibrated at 37°C for 15 min before use. Warming solution (WS) 1 consisted of 7 ml HM and 3 ml of SM, WS2 consisted of 7.5 ml of HM and 2.5 ml of SM, WS3 had 8.5 ml of HM and 1.5 ml of SM, while WS4 consisted of 10 ml of HM. 500 µl was taken from WS1 and WS2 while 600 µl from WS3 and WS4 and placed in a 4-well nunc dish which was calibrated at 37°C for 15 min before warming. Blastocysts were warmed by removing the fibre plug and placing the straw containing the blastocyst in a well with WS 1. Blastocysts were then immediately placed in WS 2 for 5 min, then into WS 3 for 5 min and then into WS 4 for 5 min after which they were placed into pre-equilibrated Cleave Media. Prior to transfer, blastocysts were cultured for 3-4 h in Cleave Media (Cook Medical Pty Ltd, QLD, Australia) to allow them to re-expand in order to determine survival rate before being transferred.

### Pseudopregnancy and embryo transfer

Swiss female mice aged between 8-12 weeks were used as recipient mothers for embryo transfer. These were placed with vasectomised adult C57BI6 x CBA FI males in order to induce pseudopregnancy. On day 2.5 dpc blastocysts from control and 10 ng/ml GM-CSF groups were surgically transferred to contralateral uterine horns (Zander-Fox DL et al., 2015, Reduction of mitochondrial function by FCCP during mouse cleavage stage embryo culture reduces birth weight and impairs the metabolic health of offspring, Biology of Reproduction, 92: 124). Six re-expanded blastocysts from each group were transferred to one horn chosen at random. A total of 11 transfers were undertaken for each treatment group. Mice were culled on day 17.5 of pregnancy and the number of implantations and foetuses determined. Fetal weight, crown rump length and diameter and placental weight were also determined.

### RESULTS

### The addition of GM-CSF to defined oocyte maturation media increases on time blastocyst development and blastocyst rate in mice

Mouse oocytes derived from hormonally stimulated prepubertal mice were matured in oocyte maturation media with or without supplemented GM-CSF. The effect of GM-CSF on subsequent embryo development is shown in Table 7.

**TABLE 7**

| **Effect of GM-CSF on murine embryo development** | | | | |
|---|---|---|---|---|
| **GM-CSF (ng/ml)** | **n** | **Cleaved** | **Day 4 Blastocyst** | **Day 5 Blastocyst** |
| 0 | 223 | 87.3(13.9) | 13.8 (2.1) | 29.5 (4.3) |
| 2 | 251 | 89.0 (7.4) | 19.4 (4.1) | 38.0 (4.3) |
| 10 | 226 | 88.4 (7.0) | 24.6 (3.6) | 36.1 (4.5) |

| | | | | |
|---|---|---|---|---|
| Values are percentage of total (n) and are mean ± standard error (SEM) of 6 replicates | | | | |

The addition of 2 ng/ml and 10 ng/ml of GM-CSF significantly increased the number of blastocysts present on day 4 of embryo culture by 41% and 78%, respectively. This is the stage that blastocysts would normally form *in vivo* demonstrating that the addition of GM-CSF to maturation media can overcome the developmental delay seen when embryos are produced and/or cultured *in vitro* to increase on-time blastocyst development. The addition of 2 ng/ml and 10 ng/ml GM-CSF to oocyte maturation media also increased the total number of blastocysts present at the end of culture on day 6 by 28% and 22%, respectively.

### The addition of GM-CSF to maturation media increases blastocyst cell numbers in mice

The blastocysts above were differentially stained to determine inner cell mass, trophectoderm and total cell numbers. The results are shown in Figure 2. The addition of 2 ng/ml and 10 ng/ml of murine GM-CSF to oocyte maturation media increased blastocyst inner cell mass by 20.5% and 52.1%, trophectoderm cell number by 18.2% and 25.0 %, and total cell number by 18.8% and 32.9%, respectively.

### The addition of GM-CSF to oocyte maturation media reduces the incidence of DNA damage in murine blastocysts

The incidence of DNA damage was determined in murine *in vitro* produced blastocysts by examining the incidence of γH2AX staining using immunohistochemistry in control and blastocysts produced using oocytes matured in maturation media containing 2 ng/ml and 10 ng/ml of GM-CSF. The results are shown in Figure 3. The addition of GM-CSF reduced the proportion of positive cells in the 2 ng/ml and 10 ng/ml groups by 8.4% and 29.1%, respectively compared with the control.

### The addition of GM-CSF to oocyte maturation media increases pregnancy and implantation rates in mice

Control and blastocysts produced using oocytes matured in maturation media containing 10 ng/ml of GM-CSF were vitrified and warmed. Vitrified blastocysts which expanded following warming were transferred to contralateral (opposite) uterine horns chosen at random of recipient mice. Mice were sacrificed on day 17.5 post coitus and the number of implantations and foetuses determined.

Pregnancy rate as determined by the number of horns which had implantations/foetuses present was increased by 50% in the GM-CSF group. As shown in Figure 4, implantation rate was increased by 64.7% in the GM-CSF group.

### The addition of GM-CSF to oocyte maturation media increases birth rate in mice

Control and blastocysts produced using oocytes matured in media containing 10ng/ml GM-CSF were vitrified and warmed. Vitrified blastocysts which expanded following warming were transferred to contralateral or opposite horns of recipient mice and the number of fetuses present on day 17.5 were used to measure birth rate. As shown in Figure 5, birth rate was increased in the GM-CSF group by 25% compared with the control group.

### SUMMARY

The results from Examples 1 to 3 show that the addition of GM-CSF to oocyte maturation media can increase oocyte developmental competence. This results in marked improvement in embryo quantity and quality, which in turn increases implantation and birth rates. In particular, the present invention demonstrates the surprising and unexpected findings that the addition GM-CSF to oocyte maturation media:
- Increases the number of oocytes that develop to the blastocyst stage following fertilisation;
- Increases the number of oocytes that reach the blastocyst stage at the same time as that would occur normally *in vivo* (on-time blastocyst rate) following fertilisation. Embryos produced and/or cultured are delayed in their development compared with their *in vivo* counterparts highlighting deficiencies of current *in vitro* maturation fertilisation and culture systems;
- Increases the number of blastocysts that hatch. As well as a measure of on-time embryo development, the ability of a blastocyst to hatch from the zona pellucida is used as a measure of embryo viability;
- Increases the number of blastocyst inner cell mass cells. The inner cell mass comprises the cells which give rise to the fetus and has been shown previously to correlate with implantation rate.
- Increases the number of trophectoderm cells blastocysts contain. These are the cells which contribute to the placenta and as such effects placental growth and development which in turn influences fetal growth and development;
- Increases the total number of cells blastocysts contain;
- Reduces the incidence of DNA damage in blastocysts demonstrating that the addition of GM-CSF to maturation media reduces the environmental stress associated with *in vitro* culture;
- Increases the number of recipients or surrogates which become pregnant/pregnancy rate following embryo transfer;
- Increases the number of cryopreserved blastocysts which implant; and
- Increases the number of cryopreserved blastocysts which develop to term.

### EXAMPLE 4

### Effect of GM-CSF on Maturation of Oocytes and Embryos in vitro

The effect that the presence of GM-CSF in a culture medium has on blastocyst development when an oocyte and an embryo derived from the oocyte are cultured in the medium is examined. In effect, it can be determined whether GM-CSF has an additive or even synergistic effect when used in maturation culture media from the oocyte through to the embryo stage.

Oocytes are matured *in vitro* in media containing GM-CSF according to the methods set out above in Examples 1 to 3. The matured oocytes are then fertilised according to the methods described in Examples 1 to 3 and cultured in culture media with or without GM-CSF present in the culture media.

Fertilisation rate and the number of blastocyst stage embryos present, together with ICM, trophectoderm and total cell numbers are examined as per Examples 1 to 3 to determine whether the addition of GM-CSF to maturation and culture media has an additive or even synergistic effect on blastocyst development. Experiments are replicated a number of times to confirm the added benefit of using GM-CSF media in both the oocyte maturation, fertilisation, and embryo stage culturing of the IVF process.

With respect to pigs, as shown in Table 8, the inventor has demonstrated that the addition of 2 ng/ml and 10 ng/ml of GMCS-F to oocyte maturation media alone increased cleavage rate by 15.7% and 14.4%, respectively. However, the addition of 10 ng/ml of GMCSF to maturation, fertilisation and culture media increased cleavage rate by 25.2% demonstrating an additive effect when GM-CSF is added throughout culture (i.e. from the oocyte through to the embryo stage).

**TABLE 8**

| **Effect of GM-CSF on porcine embryo development** | | | | |
|---|---|---|---|---|
| **GM-CSF (ng/ml)** | **n** | **Cleaved** | **Day 5 Blastocyst** | **Day 6 Blastocyst** |
| 0 | 96 | 66.8 (1.4) | 18.4 (4.8) | 40.7 (0.2) |
| 2 | 96 | 77.3 (9.9) | 18.7 (3.7) | 43.8 (0.9) |
| 10 | 89 | 76.4 (0.8) | 34.8 (0.7) | 48.9 (7.3) |
| 10 + 10 +10 | 92 | 83.7 (0.4) | 27.9 (7.5) | 52.3 (2.2) |

| | | | | |
|---|---|---|---|---|
| Values are percentage of total (n) and are mean ± standard error (SEM) of 2 replicates | | | | |

Furthermore, as shown in Table 8, the addition of 2 ng/ml and 10 ng/ml of GM-CSF to maturation media alone increased day 6 blastocyst rate by 7.6% and 20.1%, respectively. However, the addition of 10 ng/ml of GM-CSF to maturation, fertilisation and culture media increased day 6 blastocyst rate by 28.5% demonstrating an additive effect when GM-CSF is added throughout culture.

## Claims

1. A method of increasing maturation of a mammalian oocyte *in vitro,* the method comprising culturing the oocyte *in vitro* in a mammalian oocyte maturation medium comprising 0.1 ng/ml to 100 ng/ml species-specific granulocyte macrophage-colony stimulating factor (GM-CSF).

2. The method of claim 1, wherein the GM-CSF increases developmental competence of the oocyte.

3. The method of claim 1 or 2, wherein the mammalian oocyte is selected from the group consisting of a human oocyte, a bovine oocyte, a porcine oocyte, an equine oocyte, a canine oocyte, a feline oocyte, a murine oocyte, an ovine oocyte, and a non-human primate oocyte.

4. The method of any one of claims 1 to 3, wherein the mammalian oocyte is from an aged and/or obese subject.

## Patentansprüche

1. Verfahren zur Förderung der Reifung einer Säugetier-Eizelle *in vitro*, wobei das Verfahren ein Kultivieren der Eizelle *in vitro* in einem Reifungsmedium für Säugetier-Eizellen umfasst, das 0,1 ng/ml bis 100 ng/ml artspezifischen Granulozyten-Makrophagen-Kolonie-stimulierenden Faktor (GM-CSF) umfasst.

2. Verfahren nach Anspruch 1, wobei der GM-CSF die Entwicklungskompetenz der Eizelle fördert.

3. Verfahren nach Anspruch 1 oder 2, wobei die Säugetier-Eizelle ausgewählt ist aus der Gruppe, bestehend aus einer menschlichen Eizelle, einer Rinder-Eizelle, einer Schweine-Eizelle, einer Pferde-Eizelle, einer Hunde-Eizelle, einer Katzen-Eizelle, einer Mäuse-Eizelle, einer Schaf-Eizelle und einer Eizelle eines nicht-menschlichen Primaten.

4. Verfahren nach einem jeglichen der Ansprüche 1 bis 3, wobei die Säugetier-Eizelle von einem älteren und/oder übergewichtigen Lebewesen stammt.

## Revendications

1. Procédé d'augmentation de la maturation d'un ovocyte de mammifère *in vitro,* le procédé comprenant la culture de l'ovocyte *in vitro* dans un milieu de maturation d'ovocyte de mammifère comprenant 0,1 ng/ml à 100 ng/ml de facteur de stimulation des colonies macrophages-granulocytes (GM-CSF) spécifique de l'espèce.

2. Procédé de la revendication 1, dans lequel le GM-CSF augmente la capacité de développement de l'ovocyte.

3. Procédé de la revendication 1 ou 2, dans lequel l'ovocyte de mammifère est choisi dans le groupe constitué d'un ovocyte humain, d'un ovocyte bovin, d'un ovocyte porcin, d'un ovocyte équin, d'un ovocyte canin, d'un ovocyte félin, d'un ovocyte murin, d'un ovocyte ovin et d'un ovocyte de primate non humain.

4. Procédé de l'une quelconque des revendications 1 à 3, dans lequel l'ovocyte de mammifère provient d'un sujet âgé et/ou obèse.
